# EUROPEAN PATENT APPLICATION

(11) **EP 4 667 580 A1**
(43) Date of publication of application: **24.12.2025**
(21) Application number: 24757002.1
(22) Date of filing: 16.02.2024
(51) Int. Cl.: C12P 21/02, C07K 19/00, C12N 1/21, C12N 9/04, C12N 15/53

(54) **METHOD FOR SECRETORY PRODUCTION OF PROTEINS**

(30) Priority: 16.02.2023 JP 2023022171
(71) Applicant: AJINOMOTO CO., INC., Chuo-ku Tokyo 104-8315 (JP)
(72) Inventor: NAGASE, Yumi, Kawasaki-shi, Kanagawa 210-8681 (JP); MATSUDA, Yoshihiko, Kawasaki-shi, Kanagawa 210-8681 (JP)
(74) Representative: Hoffmann Eitle
(86) International application number: PCT/JP2024/005612
(87) International publication number: WO 2024/172163

(57) **Abstract**

A novel technique for improving secretory production of a heterologous protein by coryneform bacteria is provided, and thereby a method for secretory production of a heterologous protein is provided. A coryneform bacterium having an ability of secretory producing a heterologous protein and having been modified so that an activity of a Mdh protein is reduced is cultured to produce the heterologous protein by secretory production.

## Description

### Technical field

The present invention relates to a method for secretory production of a heterologous protein.

### Background Art

Secretory production of heterologous proteins by micro-organisms has been reported to date, including secretory production of heterologous proteins by bacteria of the genus Bacillus (Non-patent document 1), methanol-utilizing yeast Pichia pastoris (Non-patent document 2) and filamentous fungi of the genus Aspergillus (Non-patent document 3 and 4).

Attempts have also been made to secretively produce heterologous proteins using corynebacteria. As for secretory productions of heterologous proteins using coryneform bacteria, secretion of a nuclease and lipase by Corynebacterium glutamicum (hereafter referred to as C. glutamicum)(Patent document 1, Non-Patent document 5), secretion of a protease such as satylysin (Non-Patent document 6), secretion of a protein using signal peptides of cell surface proteins PS1 and PS2 (also called CspB) of coryneform bacteria (Patent document 2), secretion of a fibronectin-binding protein using the signal peptide of PS2 (CspB) (Non-Patent document 7), secretion of a protransglutaminase using signal peptides of PS2 (CspB) and SlpA (also called CspA) (Patent document 3), secretion of a protein using a mutant secretion system (Patent document 4), secretion of a protransglutaminase by a mutant strain (Patent document 5), and others have been reported. In addition, techniques to increase the secretory production of heterologous proteins by coryneform bacteria include reducing the activity of a cell surface protein (Patent documents 6 and 7), reducing the activity of a penicillin-binding protein (Patent document 6), enhancing the expression of a gene encoding a metallopeptidase (Patent document 7 ), introducing a mutation into a ribosomal protein S1 gene (Patent document 8), inserting an amino acid sequence including Gln-Glu-Thr between the signal peptide and the heterologous protein and expressing the heterologous protein (Patent document 9).

A common protein secretion pathway is a pathway called "Sec system", which is widespread in prokaryotes and eukaryotes, but recently a protein secretion pathway completely different from the Sec system has been found in chloroplast thylakoid membranes in plant cells (Non-Patent document 8). This novel secretion pathway has been named the "Tat system" (Twin-Arginine Translocation system) because of the common presence of arginine-arginine sequences in the signal sequence of a protein secreted by it (Non-Patent document 8). It is known that, in the Sec system, proteins are secreted in a state before forming higher-order structures, whereas in the Tat system, proteins are secreted through a plasma membrane after forming higher-order structures in the cell (Non-Patent document 9). Secretory production of proteins using a Tat-dependent signal peptide has also been reported in coryneform bacteria (Patent document 8 and 10).

Malate dehydrogenase is a redox enzyme that catalyzes the conversion of malic acid to oxaloacetic acid and/or the reverse reaction. Like many other bacteria, C. glutamicum has two types of malate dehydrogenase: a cytoplasmic malate dehydrogenase (Mdh) encoded by a mdh gene and a transmembrane malate: quinone-oxidoreductase (Mqo) encoded by a mqo gene, and these two malate dehydrogenases function in concert. Under a normal culture condition, Mqo is responsible for most of the reaction that converts malate to oxaloacetic acid, while Mdh functions only at low concentrations of oxaloacetic acid and high concentrations of malate. For C. glutamicum, a mqo-gene-deficient strain cannot grow under a minimal culture condition, but a mdh-gene-deficient strain grows as well as the wild strain, suggesting that Mqo can compensate for the function of Mdh (Non-patent document 10).

There are several findings on the relationship between the mdh gene and substance production in C. glutamicum, as shown below. Namely, increased isobutanol production ability due to loss of the mdh gene (Non-patent document 11), increased L-amino acid production ability due to enhanced Mdh activity (Patent document 11) and increased succinic acid production ability due to enhanced Mdh activity (Patent document 12) are known.

However, the relationship between Mdh and the secreted production of heterologous proteins in coryneform bacteria has not been reported to date.

### Prior art documents

### Patent documents

[Patent document 1] US Patent No. 4,965,197.
[Patent document 2] JP H6-502548 A.
[Patent document 3] Japanese Patent No. 4320769.
[Patent document 4] JP H11-169182 A.
[Patent document 5] Japanese Patent No. 4362651.
[Patent document 6] WO2013/065869.
[Patent document 7] WO2013/065772.
[Patent document 8] WO2013/118544.
[Patent document 9] WO2013/062029.
[Patent document 10] Japanese Patent No. 4730302.
[Patent document 11] JP 2003-144161 A.
[Patent document 12] Japanese Patent No. 4760121.

### Non-patent documents

[Non-patent document 1] Microbiol. rev. 57, 109-137 (1993).
[Non-patent document 2] Biotechnol. 11, 905-910 (1993).
[Non-patent document 3] Biotechnol. 6, 1419-1422 (1988).
[Non-patent document 4] Biotechnol. 9, 976-981 (1991).
[Non-patent document 5] J. Bacteriol. 174, 1854-1861 (1992).
[Non-patent document 6] Appl. Environ. Microbiol. 61, 1610-1613 (1995).
[Non-patent document 7] Appl. Environ. Microbiol. 63, 4392-4400 (1997).
[Non-patent document 8] EMBO J., 14, 2715-2722 (1995).
[Non-patent document 9] J. Biol. Chem. 25;273(52), 34868-74 (1998).
[Non-patent document 10] J. Bacteriol. 182, 6884-6891 (2000).
[Non-patent document 11] Appl. Environ. Microb. 77, 3300-3310 (2011).

### Summary of Invention

### Technical Problem

An aspect of the present invention is to provide a novel technique to improve secretory production of a heterologous protein by a coryneform bacterium and to provide a method for secretory production of a heterologous protein by a coryneform bacterium.

### Solution to Problem

The inventors conducted intensive research to solve the above problem. As a result, they found that a secretory production ability of a coryneform bacterium to produce a heterologous proteins can be improved by modifying the coryneform bacterium so that an activity of a Mdh protein is reduced, and accomplished the present invention.

In other words, the present invention can be exemplified as follows.
[1] A method for producing a heterogeneous protein, comprising:
   culturing a coryneform bacterium having a genetic construct for a secretory expression of the heterologous protein; and
   collecting the heterologous protein secretory produced,
   wherein said coryneform bacterium has been modified so that an activity of a Mdh protein is reduced as compared to an unmodified bacterium,
   wherein the genetic construct contains, in the direction from 5' to 3', a promoter sequence that functions in the coryneform bacteria, a nucleic acid sequence encoding a signal peptide that functions in the coryneform bacteria, and a nucleic acid sequence encoding the heterologous protein, and
   wherein the heterologous protein is expressed as a fusion protein with the signal peptide.
[2] The method as described above (specifically in [1]), wherein the Mdh protein is a protein as described in (a), (b) or (c) below:
   (a) a protein comprising the amino acid sequence of SEQ ID NO 40;
   (b) a protein comprising the amino acid sequence of SEQ ID NO 40 but which includes a substitution(s), a deletion(s), an insertion(s) and/or an addition(s) of 1 to 10 amino acid residues, and having malate dehydrogenase activity; and
   (c) a protein comprising an amino acid sequence having 90% or more identity to the amino acid sequence of SEQ ID NO 40 and having the malate dehydrogenase activity.
[3] The method as described above (specifically in [1] or [2]), wherein the activity of the Mdh protein is reduced by decreasing the expression of a mdh gene or by disrupting a mdh gene.
[4] The method as described above (specifically, [1] to [3]) wherein the activity of the Mdh protein is reduced due to a deletion of part or all of the amino acid sequence of the Mdh protein.
[5] The method as described above(specifically in [4]) wherein at least the region corresponding to positions 313 to 328 of SEQ ID NO 40 in the amino acid sequence of the Mdh protein is deleted.
[6] The method as described above (specifically in [4]) wherein at least 16 residues at the C-terminus of the amino acid sequence of the Mdh protein are deleted.
[7] The method as described above (specifically, any of [4]~ [6]), wherein the deletion was caused by deletion of part or all of the coding region of the mdh gene, an introduction of a stop codon into the coding region of the mdh gene, a frameshift in the coding region of the mdh gene, or a combination thereof.
[8] The method as described above (specifically, any of [1] to [7]), wherein the coryneform bacterium is further modified to harbor a phoS gene encoding a mutated PhoS protein.
[9] The method as described above (specifically as described in [8]), wherein the mutation is a replacement of an amino acid residue corresponding to the tryptophan residue at position 302 of SEQ ID NO 2 in the wild-type PhoS protein with an amino acid residue other than aromatic amino acids and histidine residues.
[10] The method as described above (specifically as described in [9]), wherein the amino acid residue other than aromatic amino acids and histidine residues is a lysine residue, alanine residue, valine residue, serine residue, cysteine residue, methionine residue, aspartic acid residue or aspartic residue.
[11] The method as described above (specifically as described in [9] or [10]), wherein the wild-type PhoS protein is a protein as described in (a), (b) or (c) below:
   (a) a protein comprising any of the amino acid sequences of SEQ ID NOs 2 to 7;
   (b) a protein comprising any of the amino acid sequences of SEQ ID NOs 2 to 7, but which includes a substitution(s), a deletion(s), an insertion(s) and/or an addition(s) of 1 to 10 amino acid residues, and having a function as a sensor kinase of the PhoRS system; and
   (c) a protein comprising an amino acid sequence having 90% or more identity to any of the amino acid sequences of SEQ ID NOs 2 to 7 and having a function as a sensor kinase of the PhoRS system.
[12] The method as described above (specifically, any of [1] to [11]), wherein the signal peptide is a Tat-dependent signal peptide.
[13] The method as described above (specifically, [12]), wherein the Tat-dependent signal peptide is selected from the group consisting of a TorA signal peptide, SufI signal peptide, PhoD signal peptide, LipA signal peptide and IMD signal peptide.
[14] The method as described above (specifically as described in [12] or [13]), wherein the coryneform bacterium is further modified so that the expression of one or more genes encoding a Tat secretion system is increased compared to an unmodified bacterium.
[15] The method as described above (specifically in [14]), wherein the genes encoding the Tat secretion system comprise a tatA gene, tatB gene, tatC gene, and tatE gene.
[16] The method as described above (specifically, any of [1] to [11]), wherein the signal peptide is a Sec-dependent signal peptide.
[17] The method as described above (specifically as described in [16]), wherein the Sec-dependent signal peptide is a signal peptide selected from the group consisting of a PS1 signal peptide, PS2 signal peptide, and SlpA signal peptide.
[18] The method as described above (specifically, any of [1] to [17]), wherein the genetic construct further comprises a nucleic acid sequence encoding an amino acid sequence comprising Gln-Glu-Thr between the nucleic acid sequence encoding the signal peptide that functions in the coryneform bacteria and the nucleic acid sequence encoding the heterologous protein.
[19] The method described above (specifically in [18]) wherein the genetic construct further comprises a nucleic acid sequence encoding an amino acid sequence used for an enzymatic cleavage between the nucleic acid sequence encoding the amino acid sequence including Gln-Glu-Thr and the nucleic acid sequence encoding the heterologous protein.
[20] The method described above (specifically, any of [1] to [19]), wherein the coryneform bacterium belongs to the genus *Corynebacterium.*
[21] The method described above (specifically in [20]), wherein the coryneform bacterium is *Corynebacterium glutamicum.*
[22] The method described above (specifically in [21]), wherein the coryneform bacterium is a modified strain derived from *Corynebacterium glutamicum* AJ12036 (FERM BP-734) or a modified strain derived from *Corynebacterium glutamicum* ATCC 13869.
[23] The method (specifically, any of [1] to [22]), wherein the coryneform bacterium is a coryneform bacterium having a reduced number of molecules per cell of cell surface proteins as compared to the unmodified bacterium.

### Effect of the invention

The present invention allows for the efficient secretory production of heterologous proteins.

### Brief Description of Drawings

Fig. 1 is a photograph showing SDS-PAGE results of Protein L (antibody-binding domain of Protein L fused with the signal peptide of CspA) expression in C. glutamicum YDK010::phoS (W302C) strain and its mdh gene deletion strain.
Fig. 2 is a photograph showing SDS-PAGE results of Protein L (antibody-binding domain of Protein L fused with the signal peptide of CspA) expression in C. glutamicum YDK010::phoS (W302C) strain and its mdh gene stop codon insertion strain.
Fig. 3 is a photograph showing SDS-PAGE results of CspB6Xa-LFABP (LFABP fused with the signal peptide of CspB, the N-terminal sequence of mature CspB and the Factor Xa protease recognition sequence) expression in C. glutamicum YDK010::phoS (W302C) strain and its mdh gene stop codon insertion strain.
Fig. 4 is a photograph showing SDS-PAGE results of PTG (protransglutaminase fused with CspA signal peptide) expression in C. glutamicum YDK010::phoS (W302C) strain and its mdh gene stop codon insertion strain.
Fig. 5 is a photograph showing SDS-PAGE results of PTG (protransglutaminase fused with TorA signal peptide) expression in C. glutamicum YDK010::phoS (W302C) strain and its mdh gene stop codon insertion strain.

### Detailed Description of Exemplary Embodiments

Hereinafter, the present invention will be explained in detail.

The method of the present invention relates to a method for producing a heterologous protein by culturing a coryneform bacterium having a genetic construct for secretory expression of the heterologous protein, and collecting the secretion-produced heterologous protein, wherein the coryneform bacterium is modified to have a reduced activity of a Mdh protein.

### <1> Coryneform bacterium used in the method of the present invention.

The coryneform bacterium used in the method of the present invention is a coryneform bacterium having a genetic construct for secretory expression of a heterologous protein and modified to reduce the activity of a Mdh protein. The coryneform bacterium used in the method of the present invention is also referred to as "bacterium of the present invention" or "coryneform bacterium of the present invention". The genetic construct for secreted expression of a heterologous protein possessed by the bacterium of the present invention is also referred to as "genetic construct used in the present invention". Also, the bacterium of the present invention or the strain used to construct it is also referred to as the "host".

### <1-1> Coryneform bacterium having an ability to secrete and produce a heterologous protein

The coryneform bacterium of the present invention is capable of secretory production of a heterologous protein. The coryneform bacterium of the present invention has an ability to produce a heterologous protein in secretory production, relying at least on having a genetic construct for secretory expression of the heterologous protein (the genetic construct used in the present invention). The coryneform bacterium of the present invention may have an ability to produce a heterologous protein in secretory production, specifically by having a genetic construct for secretory expression of the heterologous protein, or by a combination of having a genetic construct for secretory expression of the heterologous protein and other characteristic(s). Other characteristic(s) include a modification(s) that reduce the activity of Mdh protein and other characteristic(s) as described below.

In the present invention, the term "secretion" of a protein means that the protein is transported outside a bacterial cell (extracellularly transported). Examples of a position outside of a bacterial cell (outside of a cell) include a culture medium and a surface layer of the cell. This means that molecules of the secreted protein may, for example, be present in the culture medium, on the cell surface layer, or both in the culture medium and on the cell surface layer. In other words, the "secretion" of a protein is not limited to when all the molecules of the protein are completely free in the medium, but also includes when, for example, all the molecules of the protein are present in the surface layer of the cell, or where some molecules of the protein are present in the medium and the rest are present in the surface layer of the cell.

That is, in the present invention, the term "ability to produce a heterologous protein by secretory production" refers to an ability of the bacterium of the present invention to secrete the heterologous protein into a medium and/or a cell surface layer, and accumulate it there to such an extent that the heterologous protein can be collected from the medium and/or the cell surface layer, when the bacterium is cultured in the medium. The accumulation amount may be, for example, in terms of the accumulation amount in the medium, preferably 10 µg/L or more, more preferably 1 mg/L or more, particularly preferably 100 mg/L or more, still more preferably 1 g/L or more. Also, the accumulation amount may be, for example, in terms of the accumulation amount in the cell surface layer, such an amount that if the heterologous protein in the cell surface layer is collected and suspended in a liquid of the same volume as the medium, the concentration of the heterologous protein in the suspension is preferably 10 µg/L or more, more preferably 1 mg/L or more, particularly preferably 100 mg/L or more. In addition, in the present invention, the term "protein" to be secretory produced refers to a concept also including those called a peptide, such as oligopeptides and polypeptides.

In the present invention, the term "heterologous protein" refers to an exogenous protein relative to a coryneform bacterium that expresses and secretes that protein. The heterologous protein may be, for example, a protein derived from a microorganism, a protein derived from a plant, a protein derived from an animal, a protein derived from a virus, or even a protein of which the amino acid sequence is artificially designed. The heterologous protein may in particular be a protein derived from human. The heterologous protein may be a monomeric protein or a multimeric protein. The term "multimeric protein" refers to a protein that may exist as a multimer having two or more subunits. In the multimer, the subunits may be linked by covalent bonds such as disulfide bonds, linked by non-covalent bonds such as hydrogen bonds and hydrophobic interaction, or linked by a combination thereof. The multimer preferably includes one or more intermolecular disulfide bonds. The multimer may be a homo-multimer consisting of a single kind of subunit, or may be a hetero-multimer having two or more kinds of subunits. In the case where the multimeric protein is a hetero-multimer, it is sufficient that at least one subunit selected from the subunits constituting the hetero-multimer is a heterologous protein. That is, all the subunits may be heterologous, or only a part of subunits may be heterologous. Although the heterologous protein may be a secretory protein in nature, or may be a non-secretory protein in nature, it is preferably a secretory protein in nature. Furthermore, the heterologous protein may be a Tat-dependent secretory protein in nature, or may be a Sec-dependent secretory protein in nature. Specific examples of the "heterologous protein" will be mentioned later.

The heterologous protein to be produced may have a single kind of protein, or two or more kinds of proteins. Moreover, when the heterologous protein is a hetero-multimer, only one kind of subunit may be produced, or two or more kinds of subunits may be produced. That is, the term "secretory production of a heterologous protein" includes secretory production of all the subunits constituting an objective heterologous protein, as well as secretory production of only a part of the subunits constituting an objective heterologous protein.

Coryneform bacteria are aerobic gram-positive bacilli. Examples of the coryneform bacteria include *Corynebacterium* bacteria, *Brevibacterium* bacteria, *Microbacterium* bacteria, and so forth. Advantages of use of the coryneform bacteria include that they inherently secrete an extremely small amount of proteins out of cells compared with fungi, yeasts, *Bacillus* bacteria, *etc.,* which are conventionally used for secretory production of proteins, and therefore the purification process of a heterologous protein secretory produced is expected to be simplified or eliminated, that they can grow well in a simple medium containing a saccharide, ammonia, mineral salts, etc., and therefore they are excellent in view of cost of medium, culture method, and culture productivity, and so forth.

Specific examples of coryneform bacteria include the following species:
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens*)
*Corynebacterium herculis*
*Brevibacterium divaricatum* (*Corynebacterium glutamicum*)
*Brevibacterium flavum* (*Corynebacterium glutamicum*)
*Brevibacterium immariophilum*
*Brevibacterium lactofermentum* (*Corynebacterium glutamicum*)
*Brevibacterium roseum*
*Brevibacterium saccharolyticum*
*Brevibacterium thiogenitalis*
*Corynebacterium ammoniagenes* (*Corynebacterium stationis*)
*Brevibacterium album*
*Brevibacterium cerinum*
*Microbacterium ammoniaphilum*

Specific examples of coryneform bacteria include the following strains:
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium thermoaminogenes* (*Corynebacterium efficiens*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The *Corynebacterium* bacteria include bacteria that had previously been classified into the genus *Brevibacterium,* but have since and are currently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but are presently re-classified into *Corynebacterium stationis* on the basis of nucleotide sequence analysis of 16S rRNA etc. (Int. J. Syst. Evol. Microbiol., 60, 874-879 (2010)).

These strains are available from, for example, the American Type Culture Collection (Address: 12301 Parklawn Drive, Rockville, Maryland 20852, P.O. Box 1549, Manassas, VA 20108, United States of America). That is, registration numbers are assigned to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

In particular, the *Corynebacterium glutamicum* (*C. glutamicum*) AJ12036 strain (FERM BP-734), which was isolated as a streptomycin (Sm) resistant mutant strain from a wild-type strain *C. glutamicum* ATCC 13869 is predicted to have a mutation in a gene responsible for a function involved in secretion of proteins, and shows an extremely high secretory production ability for proteins as high as about 2 to 3 times in terms of accumulation amount of proteins under optimum culture conditions, compared with the parent strain (wild-type strain), and therefore it is preferred as a host bacterium (WO2002/081694). The AJ12036 strain was originally deposited at the Fermentation Research Institute, Agency of Industrial Science and Technology (currently, independent administrative agency, National Institute of Technology and Evaluation, International Patent Organism Depositary (NITE IPOD), #120, 2-5-8 Kazusakamatari, Kisarazu-shi, Chiba-ken, 292-0818, Japan) on March 26, 1984 as an international deposit, and assigned an accession number of FERM BP-734.

*Corynebacterium thermoaminogenes* AJ12340 (FERM BP-1539) was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on March 13, 1987 as an international deposit, and assigned an accession number of FERM BP-1539. *Brevibacterium flavum* AJ12418 (FERM BP-2205) was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on December 24, 1988 as an international deposit, and assigned an accession number of FERM BP-2205.

Moreover, a strain having an enhanced ability to produce a protein by secretory production may be selected from such a coryneform bacterium as mentioned above as a parent strain by using a mutagenesis method or a genetic recombination method and used as a host. For example, after a parent strain is treated with ultraviolet irradiation or a chemical mutation agent such as N-methyl-N'-nitrosoguanidine, a strain having an enhanced ability to produce a protein by secretory production can be selected.

Furthermore, if a strain obtained by modifying such a strain as mentioned above so that it does not produce a cell surface layer protein is used as a host, purification of the heterologous protein secreted in the medium or on the cell surface layer becomes easy, and therefore it is particularly preferred. Such modification can be carried out by introducing a mutation into the coding region of the cell surface layer protein or an expression control region thereof, on the chromosome by mutagenesis or genetic recombination. Examples of coryneform bacterium modified so that it does not produce a cell surface layer protein include the *C. glutamicum* YDK010 strain (WO2004/029254), which is a cell surface layer protein PS2 deficient strain of the *C. glutamicum* AJ12036 strain (FERM BP-734).

A coryneform bacterium having an ability of secretory production of a heterologous protein can be obtained by introducing the genetic construct used for the method of the present invention into such a coryneform bacterium as described above so as to make the bacterium harbor the genetic construct. In other words, the bacterium of the present invention can be, for example, a modified strain derived from a coryneform bacterium as described above. The bacterium of the present invention may specifically be, for example, a modified strain derived from C. glutamicum AJ12036 (FERM BP-734) or a modified strain derived from C. glutamicum ATCC 13869. The modified strain derived from C. glutamicum AJ12036 (FERM BP-734) is also a modified strain derived from C. glutamicum ATCC 13869. The genetic construct used in the present invention and the method of introduction are described below.

### <1-2> Reduction of the activity of Mdh protein.

The bacterium of the present invention is modified so that an activity of Mdh protein is reduced. The bacterium of the present invention is specifically modified so that the activity of Mdh protein is reduced compared to unmodified strains. The activity of the Mdh protein may be reduced compared to, for example, C. glutamicum AJ12036 (FERM BP-734) or C. glutamicum ATCC 13869. The bacterium of the present invention may be modified, more specifically, so that the expression of a mdh gene is reduced or the mdh gene is disrupted. Modifying the coryneform bacterium so that the activity of Mdh protein is reduced may improve the ability of the same bacterium to produce a heterologous protein by secretory production, i.e. the secretory production of a heterologous protein by the bacterium can be increased.

Bacterium of the present invention can be obtained by modifying a coryneform bacterium with an ability to produce a heterologous protein by secretory production so that the activity of Mdh protein is reduced. The bacterium of the present invention can also be obtained by modifying a coryneform bacterium so that the activity of Mdh protein is reduced and then giving it the ability to produce a heterologous protein by secretory production. In the present invention, a modification to construct the bacterium of the invention can be made in any order. A strain used to construct the bacterium of the present invention, before being modified to reduce the activity of Mdh protein, may or may not be capable of production of a heterologous protein by secretory production, assuming that it has a genetic construct for secretory expression of the heterologous protein. In other words, the bacterium of the present invention may, for example, have acquired the ability to produce a heterologous protein by secretory production due to being modified so that the activity of Mdh protein is reduced. Specifically, for example, the bacterium of the present invention may be derived from a strain that, before being modified to reduce the activity of Mdh protein, had a genetic construct for the secretory expression of a heterologous protein but was not capable of secretory production of a heterologous protein, and which, enabling the secretory production of a heterologous protein by being modified to reduce the activity of Mdh protein.

A Mdh protein and a mdh gene encoding it are described below. Mdh protein is a cytoplasmic malate dehydrogenase (EC 1.1.1.37). The term "malate dehydrogenase" refers to a protein (enzyme) that has the activity to catalyze the conversion of malic acid to oxaloacetic acid in the presence of an electron acceptor and/or the conversion of oxaloacetic acid to malic acid in the presence of a potential donor. The said activity is also referred to as "malate dehydrogenase activity". Examples of the electron acceptor include NAD⁺ . Examples of the potential donor include NADH.

A nucleotide sequence of the mdh gene possessed by coryneform bacterium and an amino acid sequence of a Mdh protein encoded by it can be obtained from public databases, for example, from the National Centre for Biotechnology Information (NCBI). The nucleotide sequence of the mdh gene of C. glutamicum ATCC 13869 and the amino acid sequence of the Mdh protein encoded by the gene are shown in SEQ ID NOs 39 and 40 respectively. In other words, the mdh gene may be, for example, a gene having the nucleotide sequence shown in SEQ ID NO 39. The Mdh protein may be, for example, a protein having the amino acid sequence shown in SEQ ID NO 40. The expression "having the (amino acid or nucleotide) sequence" means "including the (amino acid or nucleotide) sequence" and includes "including only of the (amino acid or nucleotide) sequence", unless otherwise stated.

The mdh gene may be a variant of the above-exemplified mdh gene (e.g. a gene having the nucleotide sequence shown in SEQ ID NO 39) as long as the original function is maintained. Similarly, the Mdh protein may be a variant of the above-exemplified Mdh protein (e.g. a protein having the amino acid sequence shown in SEQ ID NO 40) as long as the original function is maintained. Such a variant in which the original function is maintained may be referred to as "a conservative variant". In the present invention, the term "mdh gene" is not limited to the mdh gene exemplified above, but shall also encompass its conservative variant. Similarly, the term "Mdh protein" shall not be limited to the Mdh protein exemplified above, but shall encompass its conservative variant. Examples of the conservative variants include, for example, homologs and artificial modifications of the above-exemplified mdh gene and Mdh protein.

The term "original function maintained" means that a gene or protein variant has a function (e.g. activity or property) that corresponds to the function (e.g. activity or property) of the original gene or protein. In other words, "original function is maintained" may mean, in the case of a mdh gene, that the gene variant encodes a protein (i.e. Mdh protein) in which the original function is maintained. Also, "original function maintained" may be, in the case of a Mdh protein, that the protein variant has a function as a Mdh protein (e.g. the function of a protein including the amino acid sequence shown in SEQ ID NO 40). The "original function is maintained" may also mean, in the case of a Mdh protein, that the protein variant has malate dehydrogenase activity. In other words, "function as a Mdh protein" may specifically be malate dehydrogenase activity.

Malate dehydrogenase activity (in the direction of oxaloacetic acid formation) can be measured by incubating an enzyme with a substrate (malic acid) in the presence of NAD⁺ and measuring the formation of NADH. Malate dehydrogenase activity (in the direction of malate formation) can be measured by incubating an enzyme with a substrate (oxaloacetic acid) in the presence of NADH and measuring the decrease in NADH.

The following are examples of conservative variant.

Homologs of the mdh gene or homologs of the Mdh protein can be easily obtained from public databases, for example, BLAST searches or FASTA searches using the nucleotide sequence of the above exemplified mdh gene or the amino acid sequence of the above exemplified Mdh protein as a query sequence. Homologs of the mdh gene can also be obtained, for example, by PCR using the chromosome of a coryneform bacterium as a template and using oligonucleotides made based on the nucleotide sequences of these known mdh genes as primers.

The mdh gene may be a protein having any of the amino acid sequences of the Mdh protein exemplified above (e.g. the amino acid sequence shown in SEQ ID NO 40) but which includes a substitution(s), a deletion(s), an insertion(s) and/or an addition(s) of one or several amino acid residues at one or several positions, as long as the original function is maintained. The above "one or several" may differs depending on the position of the amino acid residue in the three-dimensional structure of the protein and the type of amino acid residue, but specifically, may mean, for example, 1 to 50, 1 to 40, 1 to 30, preferably 1 to 20, more preferably 1 to 10, more preferably 1 to 5, particularly preferably 1 to 3.

The aforementioned substitution, deletion, insertion, or addition of one or several amino acid residues is a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the bacterium from which the gene is derived (mutant or variant).

The mdh gene may also be a gene encoding a protein having 80% or more, preferably 90% or more, more preferably 95% or more, even more preferably 97% or more, especially preferably 99% or more identity, to the entire amino acid sequence of the Mdh protein exemplified above (for example, the amino acid sequence shown in SEQ ID NO 40), as long as the original function is maintained.

The mdh gene may also be DNA that is able to hybridize under stringent conditions with a complementary sequence of the nucleotide sequences of the mdh gene exemplified above (e.g. the sequence shown in SEQ ID NO 39), or with a probe that can be prepared from the complementary sequence, so long as it does not have the "specific mutation" and the original function thereof is maintained. The term "stringent conditions" refers to conditions under which a so-called specific hybrid is formed, and a non-specific hybrid is not formed. Examples of the stringent conditions include those under which highly identitical DNAs hybridize to each other, for example, DNAs not less than 80% identity, preferably not less than 90% identity, more preferably not less than 95% identity, still more preferably not less than 97% identity, particularly preferably not less than 99% identity, hybridize to each other, and DNAs less identity than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe may be, for example, a part of a sequence that is complementary to the gene as described above. Such a probe can be prepared by PCR using oligonucleotides prepared based on of the nucleotide sequences of known genes as primers and a DNA fragment containing any of these nucleotide sequences as a template. As the probe, for example, a DNA fragment having a length of about 300 bp can be used. In such a case, the washing conditions of the hybridization may be, for example, 50°C, 2 x SSC and 0.1% SDS.

The mdh gene may also have a nucleotide sequence in which any codon is replaced by an equivalent codon in the sequence of the above example mdh gene or its conservative variant.

The term "identity" between amino acid sequences means an identity between the amino acid sequences calculated by blastp with default scoring parameters (i.e., Matrix, BLOSUM62; Gap Costs, Existence=11, Extension=1; Compositional Adjustments, Conditional compositional score matrix adjustment), unless otherwise stated. The term "identity" between nucleotide sequences means an identity between the nucleotide sequences calculated by blastn with default scoring parameters (i.e., Match/Mismatch Scores=1, -2; Gap Costs =Linear), unless otherwise stated.

The above description of gene and protein variants can be similarly applied to any proteins, such as PhoRS protein, cell surface protein, Tat secretion system, heterologous proteins secretory produced in the present invention, and genes encoding them.

### <1-3> Other properties.

The bacterium of the invention may have the desired properties as long as they are capable of producing a heterologous protein by secretory production. For example, the activity of a cell surface layer protein may have been reduced in the bacterium (WO2013/065869, WO2013/065772, WO2013/118544, and WO2013/062029). Also, the bacterium may have been modified so that the activity of a penicillin-binding protein is reduced (WO2013/065869). Also, the bacterium may have been modified so that the expression of a gene encoding a metallopeptidase is increased (WO2013/065772). Also, the bacterium may have been modified so as to have a mutant ribosomal protein Si gene (mutant rpsA gene) (WO2013/118544). Also, the bacterium may have been modified so as to have a mutant phoS gene (WO2016/171224). Also, the bacterium may have been modified so that the activity of a RegX3 protein is reduced (WO2018/074578). Also, the bacterium may have been modified so that the activity of a HrrSA system is reduced (WO2018/074579). Also, the bacterium may have been modified so that the activity of the Tat secretion system is increased. These characteristics or modifications may occur alone or in any appropriate combination.

### <1-3-1> Introduction of Mutant phoS Gene

The bacterium may have been modified so as to harbor a mutant phoS gene. The expression "to harbor a mutant phoS gene" is also referred to as "to have a mutant phoS gene" or "to have a mutation in a phoS gene". In addition, the expression "to harbor a mutant phoS gene" is also referred to as "to have a mutant PhoS protein" or "to have a mutation in a PhoS protein".

Hereinafter, the phoS gene and the PhoS protein will be explained. The phoS gene is a gene encoding a PhoS protein, which is a sensor kinase of the PhoRS system. The PhoRS system is one of two-component regulatory systems, and induces a response against phosphate depletion. The PhoRS system has a sensor kinase PhoS encoded by a phoS gene and a response regulator PhoR encoded by a phoR gene.

In the present invention, a PhoS protein having the "specific mutation" is also referred to as "mutant PhoS protein", and a gene encoding it is also referred to as "mutant phoS gene". The mutant phoS gene is, in other words, a phoS gene having the "specific mutation". Furthermore, in the present invention, a PhoS protein not having the "specific mutation" is also referred to as "wildtype PhoS protein", and a gene encoding it is also referred to as "wildtype phoS gene". The wildtype phoS gene is, in other words, a phoS gene not having the "specific mutation". The term "wildtype" referred to herein is used for convenience to distinguish "wildtype" ones from "mutant" ones, and "wildtype" ones are not limited to those obtained as natural substances, so long as those do not have the "specific mutation". The "specific mutation" will be described later.

Examples of the wildtype phoS gene include, for example, phoS genes of coryneform bacteria. Specific examples of the phoS genes of coryneform bacteria include, for example, the phoS genes of *C. glutamicum* YDK010, *C. glutamicum* ATCC 13032, *C. glutamicum* ATCC 14067, *C. callunae, C. crenatum,* and *C. efficiens.* The nucleotide sequence of the phoS gene of *C. glutamicum* YDK010 is shown in SEQ ID NO: 1. The amino acid sequences of the wildtype PhoS proteins encoded by these phoS genes are shown in SEQ ID NOS: 2 to 7, respectively.

The wildtype phoS gene may be a variant of any of the wildtype phoS genes exemplified above, so long as it does not have the "specific mutation" and the original function thereof is maintained. Similarly, the wildtype PhoS protein may be a variant of any of the wildtype PhoS proteins exemplified above, so long as it does not have the "specific mutation" and the original function thereof is maintained. That is, the term "wildtype phoS gene" includes not only the wildtype phoS genes exemplified above, but also includes conservative variants thereof that do not have the "specific mutation". Similarly, the term "wildtype PhoS protein" includes not only the wildtype PhoS proteins exemplified above, but also includes conservative variants thereof that do not have the "specific mutation". The aforementioned descriptions concerning conservative variants of the Mdh protein and mdh gene can be similarly applied to variants of the wildtype PhoS gene and wildtype PhoS protein. For example, the wildtype phoS gene may also be a gene encoding a protein having any of the aforementioned amino acid sequences, but which includes substitution, deletion, insertion, and/or addition of one or several amino acid residues at one or several positions, so long as it does not have the "specific mutation" and the original function thereof is maintained. Also, for example, the wildtype phoS gene may also be a gene encoding a protein having an amino acid sequence showing an identity of 80% or more, 90% or more, 95% or more, 97% or more, or 99% or more, to the total amino acid sequence of any of the aforementioned amino acid sequences, so long as it does not have the "specific mutation" and the original function thereof is maintained.

Incidentally, the expression "the original function is maintained" in reference to the wildtype PhoS may mean that a variant of the protein has a function as a PhoS protein (such as a function of a protein having any of the amino acid sequences shown in SEQ ID NOS: 2 to 7). Furthermore, the expression "the original function is maintained" used for the wildtype PhoS protein may also mean that a variant of the protein has a function as a sensor kinase of the PhoRS system. That is, the term "function as a PhoS protein" may specifically refer to a function as a sensor kinase of the PhoRS system. The term "function as a sensor kinase of the PhoRS system" may specifically refer to a function of inducing a response against phosphate depletion in the environment in combination with a response regulator PhoR protein. The term "function as a sensor kinase of the PhoRS system" may more specifically refer to a function of sensing phosphate depletion in the environment to be autophosphorylated and activating the PhoR protein via transfer of phosphate group.

Whether or not a variant of the PhoS protein has a function as a sensor kinase of the PhoRS system can be confirmed by, for example, introducing a gene encoding the variant into a phoS-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the phoS-gene-deletion strain of a coryneform bacterium, for example, a phoS-gene-deletion strain of C. *glutamicum* YDK010 or a phoS-gene-deletion strain of C. *glutamicum* ATCC 13032 can be used.

It is preferred that a histidine residue that is autophosphorylated is conserved in the wildtype PhoS protein. That is, it is preferred that a conservative mutation occurs at an amino acid residue other than the histidine residue that is autophosphorylated. The term "histidine residue that is autophosphorylated" refers to a histidine residue at position 276 of the wildtype PhoS protein. Furthermore, it is preferred that, for example, the wildtype PhoS protein has a conservative sequence of the wildtype PhoS proteins exemplified above. That is, it is preferred that a conservative mutation occurs at, for example, an amino acid residue not conserved in the wildtype PhoS proteins exemplified above.

The mutant PhoS protein has the "specific mutation" in the amino acid sequence of such a wildtype PhoS protein as described above.

That is, in other words, the mutant PhoS protein may be identical to any of the wildtype PhoS proteins exemplified above or conservative variants thereof except that the mutant PhoS protein has the "specific mutation". Specifically, the mutant PhoS protein may be, for example, a protein having any of the amino acid sequences shown in SEQ ID NOS: 2 to 7 except that the mutant PhoS protein has the "specific mutation". Specifically, the mutant PhoS protein may also be, for example, a protein having any of the amino acid sequences shown in SEQ ID NOS: 2 to 7 but including substitution, deletion, insertion, and/or addition of one or several amino acid residues, except that the mutant PhoS protein has the "specific mutation". Specifically, the mutant PhoS protein may also be, for example, a protein showing an identity of 80% or more, 90% or more, 95% or more, 97% or more, or 99% or more, to any of the amino acid sequences shown in SEQ ID NOS: 2 to 7 except that the mutant PhoS protein has the "specific mutation".

Furthermore, in other words, the mutant PhoS protein may be a variant of any of the wildtype PhoS proteins exemplified above having the "specific mutation" and further including a conservative mutation at a site other than that of the "specific mutation". Specifically, the mutant PhoS protein may be, for example, a protein having any of the amino acid sequences shown in SEQ ID NOS: 2 to 7 but having the "specific mutation", and further including substitution, deletion, insertion, and/or addition of one or several amino acid residues at a site other than that of the "specific mutation".

The mutant phoS gene is not particularly limited so long as it encodes such a mutant PhoS protein as described above.

Hereinafter, the "specific mutation" of the mutant PhoS protein will be explained.

The "specific mutation" is not particularly limited, so long as it is a mutation that changes the amino acid sequence of such a wildtype PhoS protein described above, and that is effective for secretory production a heterologous protein.

It is preferred that the "specific mutation" is a mutation that increases the secretory production amount of a heterologous protein. The expression "to increase the secretory production amount of a heterologous protein" means that a coryneform bacterium modified so as to have a mutant phoS gene (modified strain) is able to produce the heterologous protein by secretory production in an amount larger than that obtainable with an unmodified bacterium. The "unmodified bacterium" refers to a control strain not having the "specific mutation" in the phoS gene, i.e., a control strain not having any mutant phoS gene, and it may be, for example, a wildtype strain or a parent strain. Although the degree of increase meant by the expression "to produce a heterologous protein by secretory production in an amount larger than that obtainable with an unmodified bacterium" is not particularly limited so long as the secretory production amount of the heterologous protein is increased compared with that obtainable with an unmodified strain, the expression may mean that the heterologous protein is secretory produced in an amount of, for example, 1.1 times or more, 1.2 times or more, 1.3 times or more, or 2 times or more, of that obtainable with an unmodified bacterium, in terms of the accumulation amount in the medium and/or on the cell surface layer. In addition, the expression "to produce a heterologous protein by secretory production in an amount larger than that obtainable with an unmodified bacterium" may also mean that whereas the heterologous protein cannot be detected when a non-concentrated culture supernatant of an unmodified bacterium is applied to SDS-PAGE and stained with CBB, the heterologous protein can be detected when a non-concentrated culture supernatant of a modified bacterium is applied to SDS-PAGE and stained with CBB. Incidentally, the expression "to increase the secretory production amount of a heterologous protein" does not necessarily mean that the secretory production amount of every heterologous protein is increased, and it is sufficient that the secretory production amount of a heterologous protein chosen as the target of secretory production is increased. The expression "to increase the secretory production amount of a heterologous protein" may specifically mean, for example, that the secretory production amount of a heterologous protein described in the Example section is increased.

Whether a certain mutation increases the secretory production amount of a heterologous protein can be confirmed by, for example, preparing a strain modified so as to have a gene encoding the PhoS protein having the certain mutation, quantifying the amount of the heterologous protein secretory produced when the strain is cultured in a medium, and comparing it with the amount of the heterologous protein secretory produced before the modification (unmodified bacterium) is cultured in the medium.

Examples of the change of the amino acid sequence include substitution of an amino acid residue. That is, it is preferred that the "specific mutation" is replacing an amino acid residue of the wildtype PhoS protein with another amino acid residue. The amino acid residue substituted by the "specific mutation" may be one residue, or may be a combination of two or more residues. The amino acid residue substituted by the "specific mutation" may be an amino acid residue other than the histidine residue that is autophosphorylated. The amino acid residue substituted by the "specific mutation" may be an amino acid residue in the HisKA domain other than the histidine residue that is autophosphorylated. The term "histidine residue that is autophosphorylated" refers to a histidine residue at position 276 of the wildtype PhoS protein. The term "HisKA domain" refers to a region having amino acid residues at positions 266-330 of the wildtype PhoS protein. The amino acid residue substituted by the "specific mutation" may be a tryptophan residue at position 302 of the wildtype PhoS protein (W302).

In the aforementioned mutation, examples of the amino acid residue after substitution include K(Lys), R(Arg), H(His), A(Ala), V(Val), L(Leu), I(Ile), G(Gly), S(Ser), T(Thr), P(Pro), F(Phe), W(Trp), Y(Tyr), C(Cys), M(Met), D(Asp), E(Glu), N(Asn), and Q(Gln), provided that the amino acid residue after substitution is other than the original one. As the amino acid residue after substitution, for example, one resulting in improvement in the secretory production amount of a heterologous protein can be chosen.

When substitution occurs at W302, examples of the amino acid residue after substitution include amino acid residues other than aromatic amino acid and histidine residues. Specific examples of the "amino acid residues other than aromatic amino acid and histidine residues" include K(Lys), R(Arg), A(Ala), V(Val), L(Leu), I(Ile), G(Gly), S(Ser), T(Thr), P(Pro), C(Cys), M(Met), D(Asp), E(Glu), N(Asn), and Q(Gln). More specific examples of the "amino acid residues other than aromatic amino acid and histidine residues" include K(Lys), A(Ala), V(Val), S(Ser), C(Cys), M(Met), D(Asp), and N(Asn).

Incidentally, the term "specific mutation" used for the phoS gene refers to a mutation on the nucleotide sequence thereof that results in such a "specific mutation" as described above into the encoded PhoS protein.

In the present invention, the "amino acid residue at position X of the wildtype PhoS protein" refers to an amino acid residue corresponding to the amino acid residue at position X in SEQ ID NO: 2. For example, "W302" refers to an amino acid residue corresponding to the tryptophan residue at position 302 in SEQ ID NO: 2. The aforementioned positions of amino acid residues indicate relative positions, and the absolute positions thereof may shift due to deletion, insertion, addition, etc. of an amino acid residue or residues. For example, if one amino acid residue is deleted or inserted at a position on the N-terminal side of position X in the wildtype PhoS protein having the amino acid sequence shown in SEQ ID NO: 2, the amino acid residue originally at position X is relocated at position X-1 or X+1 counted from the N-terminus, however, it is still regarded as the "amino acid residue at position X of the wildtype PhoS protein". Specifically, for example, "W302" refers to the tryptophan residue at positions 302, 302, 302, 321, 275, and 286, respectively, in the amino acid sequences of wildtype PhoS proteins shown in SEQ ID NOS: 2 to 7. Furthermore, the "histidine residue at position 276 of the wildtype PhoS protein (histidine residue that is autophosphorylated)" refers to the histidine residue at positions 276, 276, 276, 295, 249, and 260, respectively, in the amino acid sequences of wildtype PhoS proteins shown in SEQ ID NOS: 2 to 7. Furthermore, the "region having amino acid residues at positions 266-330 of the wildtype PhoS protein (HisKA domain)" refers to the region having amino acid residues at positions 266-330, 266-330, 266-330, 285-349, 239-303, and 250-314, respectively, in the amino acid sequences of wildtype PhoS proteins shown in SEQ ID NOS: 2 to 7.

Incidentally, while "W302" referred to herein is typically a tryptophan residue, it may also be other than a tryptophan residue. That is, when the wildtype PhoS protein has an amino acid sequence other than the amino acid sequences shown in SEQ ID NOS: 2 to 7, "W302" can be other than a tryptophan residue. Hence, for example, the "mutation replacing W302 with a cysteine residue" includes not only a mutation, when "W302" is a tryptophan residue, for replacing this tryptophan residue with a cysteine residue, but also includes a mutation, when "W302" is K (Lys), R (Arg), H (His), A (Ala), V (Val), L (Leu), I (Ile), G (Gly), S (Ser), T (Thr), P (Pro), F (Phe), Y (Tyr), M (Met), D (Asp), E (Glu), N (Asn), or Q (Gln), for replacing this residue with a cysteine residue. The same can be similarly applied to the other mutations.

Which amino acid residue is the "amino acid residue corresponding to the amino acid residue at position X in SEQ ID NO: 2" in the amino acid sequence of an arbitrary PhoS protein can be determined by alignment between the amino acid sequence of the arbitrary PhoS protein and the amino acid sequence of SEQ ID NO: 2. The alignment can be performed by, for example, using known gene analysis software. Specific examples of such software include DNASIS produced by Hitachi Solutions, GENETYX produced by Genetyx, and so forth (Elizabeth C. Tyler et al., Computers and Biomedical Research, 24 (1) 72- 96, 1991; Barton G J et al., Journal of Molecular Biology, 198 (2), 327- 37, 1987).

The mutant phoS gene can be obtained by, for example, modifying a wildtype phoS gene so that the encoded PhoS protein has the aforementioned "specific mutation". The wildtype phoS gene to be modified can be obtained by, for example, cloning from an organism having the wildtype phoS gene, or chemical synthesis. Furthermore, the mutant phoS gene can also be obtained without using a wildtype phoS gene. For example, the mutant phoS gene may be directly obtained by chemical synthesis. The obtained mutant phoS gene may be further modified before use.

Genes can be modified by known methods. For example, an objective mutation can be introduced into a target site of DNA by the site-specific mutagenesis method. Examples of the site-specific mutagenesis method include a method of using PCR (Higuchi, R., 61, in PCR Technology, Erlich, H. A. Eds., Stockton Press (1989); Carter P, Meth. In Enzymol., 154, 382 (1987)), and a method of using a phage (Kramer, W and Frits, H. J., Meth. in Enzymol., 154, 350 (1987); Kunkel, T. A. et al., Meth. in Enzymol., 154, 367 (1987)).

Hereinafter, methods for modifying a coryneform bacterium so as to have a mutant phoS gene will be explained.

A coryneform bacterium can be modified so as to have a mutant phoS gene by introducing the mutant phoS gene into the coryneform bacterium. A coryneform bacterium can be modified so as to have a mutant phoS gene also by introducing a mutation into the phoS gene on the chromosome of the coryneform bacterium. A mutation can be introduced into a gene on a chromosome by natural mutation, mutagenesis treatment, or genetic engineering means.

Methods for introducing a mutant phoS gene into a coryneform bacterium are not particularly limited. It is sufficient that the mutant phoS gene is harbored by the bacterium so that it can be expressed under control of a promoter that functions in a coryneform bacterium. The promoter may be a promoter derived from the host, or may be a heterogenous promoter. The promoter may be the native promoter of the phoS gene, or a promoter derived from another gene. In the bacterium, the mutant phoS gene may be present on a vector that autonomously replicates out of the chromosome, such as plasmid, or may be incorporated into the chromosome. The bacterium may have only one copy of the mutant phoS gene, or two or more copies of the mutant phoS gene. The bacterium may have only one kind of mutant phoS gene, or two or more kinds of mutant phoS genes. The mutant phoS gene can be introduced, for example, in the same manner as that for introduction of a gene in methods for increasing the expression of a gene described below, or for introduction of the genetic construct used in the present invention as described below.

The bacterium may or may not have the wild type phoS gene. It is preferred that the bacterium does not have the wildtype phoS gene.

A coryneform bacterium not having the wildtype phoS gene can be obtained by disrupting the wildtype phoS gene on the chromosome. The wild type phoS gene can be disrupted by known methods. Specifically, the wildtype phoS gene can be disrupted by, for example, deleting a portion or the entire the promoter region and/or the coding region of the wildtype phoS gene.

Furthermore, by replacing the wildtype phoS gene on the chromosome with a mutant phoS gene, a coryneform bacterium modified so that it does not have the wildtype phoS gene and has the mutant phoS gene can be obtained. Examples of methods for performing such gene substitution include, for example, a method of using a linear DNA such as a method called "Red-driven integration" (Datsenko, K. A, and Wanner, B. L., Proc. Natl. Acad. Sci. USA, 97:6640-6645 (2000)), a method of utilizing the Red driven integration in combination with an excision system derived from λ phage (Cho, E. H., Gumport, R I., Gardner, J. F., J. Bacteriol., 184:5200-5203 (2002)) (refer to WO2005/010175), a method of using a plasmid including a temperature sensitive replication origin, a method of using a plasmid capable of conjugative transfer, a method of utilizing a suicide vector not including a replication origin that functions in a host (U.S. Pat. No. 6,303,383, Japanese Patent Laid-open (Kokai) No. 05-007491), and so forth.

The PhoS protein functions, that is, induces a response against phosphate depletion in the environment, in combination with a response regulator PhoR protein. Hence, the bacterium has a phoR gene so that the mutant PhoS protein functions. The phoR gene is a gene encoding a PhoR protein, which is a response regulator of the PhoRS system. The expression "to have a phoR gene" is also referred to as "to have a PhoR protein". Typically, it is sufficient that the PhoR protein inherently possessed by the bacterium functions in combination with the mutant PhoS protein. Alternatively, the bacterium may be introduced with an appropriate phoR gene, in addition to or instead of the phoR gene inherently possessed by the bacterium. The phoR gene to be introduced is not particularly limited, as long as it encodes a PhoR protein that functions in combination with the mutant PhoS protein.

Examples of the phoR gene include, for example, phoR genes of coryneform bacteria. Specific examples of the phoR genes of coryneform bacteria include, for example, the phoR genes of *C. glutamicum* YDK010, *C*. *glutamicum* ATCC 13032, *C. glutamicum* ATCC 14067, *C. callunae, C. crenatum,* and *C*. *efficiens.* The nucleotide and amino acid sequences of the phoR gene and protein of *C. glutamicum* ATCC 13032 are shown in SEQ ID NO: 8 and 9, respectively.

The phoR gene may be a variant of any of the phoR genes exemplified above, so long as the original function thereof is maintained. Similarly, the PhoR protein may be a variant of any of the PhoR proteins exemplified above, so long as the original function thereof is maintained. That is, the term "phoR gene" includes not only the phoR genes exemplified above but also includes conservative variants thereof. Similarly, the term "PhoR protein" includes not only the PhoR proteins exemplified above but also includes conservative variants thereof. The aforementioned descriptions concerning conservative variants of the Mdh protein and mdh gene can be similarly applied to variants of the phoR gene and PhoR protein. For example, the phoR gene may be a gene encoding a protein having the aforementioned amino acid sequence, but including substitution, deletion, insertion, and/or addition of one or several amino acid residues at one or several positions, so long as the original function is maintained. Also, for example, the phoR gene may also be a gene encoding a protein having an amino acid sequence having an identity of 80% or more, 90% or more, 95% or more, 97% or more, or 99% or more, to the total amino acid sequence of any of the aforementioned amino acid sequences, so long as the original function is maintained. Incidentally, the expression "the original function is maintained" used for the PhoR protein may mean that a variant of the protein has a function as a PhoR protein (such as a function of a protein having the amino acid sequence shown in SEQ ID NO: 9). Furthermore, the expression "the original function is maintained" used for the PhoR protein may also mean that a variant of the protein has a function as a response regulator of the PhoRS system. That is, the term "function as a PhoR protein" may specifically refer to a function as a response regulator of the PhoRS system. The term "function as a response regulator of the PhoRS system" may specifically refer to a function of inducing a response against phosphate depletion in the environment in combination with a sensor kinase PhoS protein. The term "function as a response regulator of the PhoRS system" may more specifically refer to a function of being activated via transfer of phosphate group from the PhoS protein that sensed phosphate depletion in the environment to be autophosphorylated, and regulating the expression of genes that respond to phosphate depletion in the environment.

Whether or not a variant of the PhoR protein functions as a response regulator of the PhoRS system can be confirmed by, for example, introducing a gene encoding the variant into a phoR-gene-deletion strain of a coryneform bacterium, and confirming whether or not responsiveness against phosphate depletion is complemented. Complementation of responsiveness against phosphate depletion can be detected, for example, as improvement of growth under phosphate depletion conditions, or as induction of the expression of genes of which the expression is known to be induced under phosphate depletion conditions (J. Bacteriol., 188, 724-732(2006)). As the phoR-gene-deletion strain of a coryneform bacterium, for example, a phoR-gene-deletion strain of *C. glutamicum* YDK010 or a phoR-gene-deletion strain of *C. glutamicum* ATCC 13032 can be used.

### <1-4-2>Reduction in Activity of Cell Surface Layer Protein

The bacterium may be a bacterium in which the activity(s) of cell surface layer protein(s) is/are reduced. Specifically, the bacterium may be a bacterium in which the activity(s) of cell surface layer protein(s) is/are reduced as compared with an unmodified bacterium. The phrase "the activity of a cell surface layer protein is reduced" may particularly mean that the number of molecules of the cell surface layer protein per cell is reduced. Hereinafter, the cell surface layer proteins and genes encoding them will be described.

The cell surface layer protein is a protein constituting the surface layer (S layer) of bacteria or archaea. Examples of cell surface layer proteins of coryneform bacteria include PS1 and PS2 (CspB) of *C. glutamicum* (Japanese

Patent Laid-open (Kohyo) No. 6-502548), and SlpA (CspA) of *C. stationis* (Japanese Patent Laid-open (Kokai) No. 10-108675). It is preferable to reduce the activity of the PS2 protein among these.

The nucleotide sequence of the cspB gene of *C. glutamicum* ATCC 13869 and the amino acid sequence of the PS2 protein (CspB protein) encoded by the gene are shown in SEQ ID NOS: 10 and 11, respectively.

Furthermore, for example, amino acid sequences of CspB homologues were reported for 28 strains of *C .glutamicum* (J. Biotechnol., 112, 177-193 (2004)). These 28 strains of *C. glutamicum* and the GenBank accession numbers of the cspB gene homologues in NCBI database are exemplified below (the GenBank accession numbers are shown in the parentheses).
*C. glutamicum* ATCC 13058 (AY524990)
*C. glutamicum* ATCC 13744 (AY524991)
*C. glutamicum* ATCC 13745 (AY524992)
*C. glutamicum* ATCC 14017 (AY524993)
*C. glutamicum* ATCC 14020 (AY525009)
*C. glutamicum* ATCC 14067 (AY524994)
*C. glutamicum* ATCC 14068 (AY525010)
*C. glutamicum* ATCC 14747 (AY525011)
*C. glutamicum* ATCC 14751 (AY524995)
*C. glutamicum* ATCC 14752 (AY524996)
*C. glutamicum* ATCC 14915 (AY524997)
*C. glutamicum* ATCC 15243 (AY524998)
*C. glutamicum* ATCC 15354 (AY524999)
*C. glutamicum* ATCC 17965 (AY525000)
*C. glutamicum* ATCC 17966 (AY525001)
*C. glutamicum* ATCC 19223 (AY525002)
*C. glutamicum* ATCC 19240 (AY525012)
*C. glutamicum* ATCC 21341 (AY525003)
*C. glutamicum* ATCC 21645 (AY525004)
*C. glutamicum* ATCC 31808 (AY525013)
*C. glutamicum* ATCC 31830 (AY525007)
*C. glutamicum* ATCC 31832 (AY525008)
*C. glutamicum* LP-6 (AY525014)
*C. glutamicum* DSM20137 (AY525015)
*C. glutamicum* DSM20598 (AY525016)
*C. glutamicum* DSM46307 (AY525017)
*C. glutamicum* 22220 (AY525005)
*C. glutamicum* 22243 (AY525006)

Since the nucleotide sequence of a gene encoding a cell surface layer protein may differ depending on species or strain to which the coryneform bacterium belongs, the gene encoding a cell surface layer protein may be a variant of any of genes encoding the cell surface layer proteins exemplified above, so long as the original function thereof is maintained. Similarly, the cell surface layer protein may be a variant of any of the cell surface layer proteins exemplified above, so long as the original function thereof is maintained. That is, the term "cspB gene" includes not only the cspB genes exemplified above but also includes conservative variants thereof. Similarly, the term "CspB protein" includes not only the CspB proteins exemplified above but also includes conservative variants thereof. The aforementioned descriptions concerning conservative variants of the Mdh protein and mdh gene can be similarly applied to variants of the cell surface layer protein and the gene encoding it. For example, the gene encoding the cell surface layer protein may be a gene encoding a protein having the aforementioned amino acid sequence, but including substitution, deletion, insertion, and/or addition of one or several amino acid residues at one or several positions, so long as the original function is maintained. Also, for example, the gene encoding the cell surface layer protein may also be a gene encoding a protein having an amino acid sequence showing an identity of 80% or more, 90% or more, 95% or more, 97% or more, or 99% or more, to the total amino acid sequence of any of the aforementioned amino acid sequences, so long as the original function is maintained. Incidentally, the expression "original function is maintained" used for the cell surface layer protein may mean that the protein has a property that if the activity of the protein is reduced in a coryneform bacterium, the secretory production amount of a heterologous protein is increased compared with that obtainable with an unmodified bacterium.

The "property that if the activity of the protein is reduced in a coryneform bacterium, the secretory production amount of a heterologous protein is increased compared with that obtainable with an unmodified bacterium" refers to a property imparting an ability to produce a heterologous protein by secretory production in an amount larger than that obtainable with an unmodified bacterium to a coryneform bacterium when the activity thereof is reduced in the coryneform bacterium. The "unmodified bacterium" refers to a control strain of which the activity(s) of cell surface layer protein(s) is/are not reduced, and it may be, for example, a wildtype strain or a parent strain. Although the degree of increase meant by the expression "to produce a heterologous protein by secretory production in an amount larger than that obtainable with an unmodified bacterium" is not particularly limited so long as the secretory production amount of the heterologous protein is increased compared with that obtainable with an unmodified bacterium, the expression may mean that the heterologous protein is secretory produced in an amount of, for example, 1.1 times or more, 1.2 times or more, 1.3 times or more, or 2 times or more, of that obtainable with an unmodified bacterium, in terms of the accumulation amount in the medium and/or on the cell surface layer. In addition, the expression "to produce a heterologous protein by secretory production in an amount larger than that obtainable with an unmodified bacterium" may also mean that whereas the heterologous protein cannot be detected when a non-concentrated culture supernatant of an unmodified bacterium is applied to SDS-PAGE and stained with CBB, the heterologous protein can be detected when a non-concentrated culture supernatant of a modified strain is applied to SDS-PAGE and stained with CBB.

Whether the activity of the protein is reduced in a coryneform bacterium, the secretory production amount of a heterologous protein is increased compared with that obtainable with an unmodified bacterium can be confirmed by preparing a strain modified so that the activity of the protein is reduced from a strain belonging to the coryneform bacteria, quantifying the secretory production amount of the heterologous protein observed when the modified strain is cultured in a medium, and comparing the quantified amount with the secretory production amount of the heterologous protein observed before being modified (un-modified strain) is cultured in the medium.

In the present invention, the expression "activity of a cell surface layer protein is reduced" includes when a coryneform bacterium has been modified so that the activity of a cell surface layer protein is reduced and a case where the activity of a cell surface layer protein is inherently reduced in a coryneform bacterium. The "case where activity of a cell surface layer protein is inherently reduced in a coryneform bacterium" includes when a coryneform bacterium is inherently deficient in a cell surface layer protein. That is, examples of a coryneform bacterium in which the activity of a cell surface layer protein is reduced include a coryneform bacterium that is inherently deficient in a cell surface layer protein. Examples of the "case where a coryneform bacterium is inherently deficient in a cell surface layer protein" include a case where a coryneform bacterium is inherently deficient in the gene encoding a cell surface layer protein. The expression "a coryneform bacterium is inherently deficient in a cell surface layer protein" may mean that a coryneform bacterium is inherently deficient in one or more proteins selected from cell surface layer protein(s) found in other strain(s) of the species to which the coryneform bacterium belongs. For example, "*C. glutamicum* is inherently deficient in a cell surface layer protein" may mean that a *C. glutamicum* strain is inherently deficient in one or more proteins selected from cell surface layer protein(s) found in other *C. glutamicum* strain(s), i.e., for example, deficient in PS1 and/or PS2 (CspB). Examples of the coryneform bacterium that is inherently deficient in a cell surface layer protein include *C. glutamicum* ATCC 13032, which is inherently deficient in the cspB gene.

### <1-3-3> Protein secretion system.

The bacterium of the present invention has a protein secretion system. The protein secretion system is not particularly limited, so long as it can secrete an objective heterologous protein. Examples of the protein secretion system include Sec system (Sec secretion system) and Tat system (Tat secretion system). The bacterium of the present invention may have been modified so that the protein secretion system is enhanced. For example, the bacterium of the present invention may have been modified so that the expression of one or more genes selected from genes encoding the Tat secretion system is increased. In the present invention, such a modification is also referred to as "enhancement of the Tat secretion system". Enhancement of the Tat secretion system is preferable particularly for cases of producing a heterologous protein by secretory production using a Tat-dependent signal peptide. Methods for increasing the expression of genes encoding the Tat secretion system are described in Japanese Patent No. 4730302.

Examples of the genes encoding the Tat secretion system include the tatA, tatB, tatC, and tatE genes.

Specific examples of the genes encoding the Tat secretion system include tatA, tatB, and tatC genes of *C. glutamicum.* The tatA, tatB, and tatC genes of *C. glutamicum* ATCC 13032 correspond to the complementary sequence of positions 1571065-1571382, the sequence of positions 1167110-1167580, and the complementary sequence of positions 1569929-1570873 in the genome sequence registered as GenBank accession NC_003450 (VERSION NC_003450.3 GI:58036263) in NCBI database, respectively. The TatA, TatB, and TatC proteins of *C. glutamicum* ATCC 13032 have been registered as GenBank accession NP_600707 (version NP_600707.1 GI:19552705, locus_tag="NCg11434"), GenBank accession NP_600350 (version NP_600350.1 GI:19552348, locus_tag="NCg11077"), and GenBank accession NP_600706 (version NP_600706.1 GI:19552704, locus_tag="NCg11433"), respectively. The nucleotide sequences of the tatA, tatB, and tatC genes of *C. glutamicum* ATCC 13032 and the amino acid sequences of the TatA, TatB, and TatC proteins of the same are shown in SEQ ID NOS: 12 to 17.

Specific examples of the genes encoding the Tat secretion system also include tatA, tatB, tatC, and tatE genes of *E. coli.* The tatA, tatB, tatC, and tatE genes of *E. coli* K-12 MG1655 correspond to the sequence of positions 4019968-4020237, the sequence of positions 4020241-4020756, the sequence of positions 4020759-4021535, and the sequence of positions 658170-658373 in the genome sequence registered as GenBank accession NC_000913(VERSION NC_000913.2 GI:49175990) in NCBI database, respectively. The TatA, TatB, TatC, and TatE proteins of *E. coli* K-12 MG1655 have been registered as GenBank accession NP_418280 (version NP_418280.4 GI:90111653, locus_tag="b3836"), GenBank accession YP_026270 (version YP_026270.1 GI:49176428, locus_tag="b3838"), GenBank accession NP_418282 (version NP_418282.1 GI:16131687, locus_tag="b3839"), and GenBank accession NP_415160 (version NP_415160.1 GI:16128610, locus_tag="b0627"), respectively.

The gene encoding the Tat secretion system may be a variant of any of the genes encoding the Tat-secretion-system exemplified above, so long as the original function thereof is maintained. Similarly, the Tat-secretion-system may be a variant of any of the Tat-secretion-systems exemplified above, so long as the original function thereof is maintained. That is, the terms "tatA gene", "tatB gene", "tatC gene", and "tatE gene" include not only the tatA, tatB, tatC, and tatE genes exemplified above, respectively, but also includes conservative variants thereof. Similarly, the terms "TatA protein", "TatB protein", "TatC protein", and "TatE protein" include not only the TatA, TatB, TatC, and TatE proteins exemplified above, respectively, but also includes conservative variants thereof. The aforementioned descriptions concerning conservative variants of the Mdh protein and mdh gene can be similarly applied to variants of the Tat-secretion-system and the gene encoding it. For example, the gene encoding the Tat-secretion-system may have any of the aforementioned amino acid sequences, but include substitution, deletion, insertion, and/or addition of one or several amino acid residues at one or several positions, so long as the original function is maintained. Also, for example, the gene encoding the Tat-secretion-system may also be a gene encoding a protein having an amino acid sequence showing an identity of 80% or more, 90% or more, 95% or more, 97% or more, or 99% or more, to the total amino acid sequence of any of the aforementioned amino acid sequences, so long as the original function is maintained. Incidentally, the expression "original function is maintained" in reference to the Tat-secretion-system may mean that the system has a function of secreting a protein fused with a Tat-dependent signal peptide at the N-terminus out of the cell.

### <1-4> Method for Reducing Activity of Protein

Hereinafter, methods for reducing the activity of a protein such as Mdh protein will be explained. The methods for reducing the activity of a protein described below can also be utilized for disruption of the wildtype PhoS protein.

The expression "the activity of a protein is reduced" means that the activity of the protein is reduced as compared with an unmodified bacterium. Specifically, the expression "the activity of a protein is reduced" means that the activity of the protein per cell is reduced as compared with that of an unmodified bacterium. The term "unmodified bacterium" used herein refers to a control strain that has not been modified so that the activity of an objective protein is reduced. Examples of the unmodified bacterium include a wildtype strain and parent strain. Specific examples of the unmodified bacterium include the respective type strains of the species of bacteria. Specific examples of the unmodified bacterium also include strains exemplified above in relation to the description of coryneform bacteria. That is, in an embodiment, the activity of a protein may be reduced as compared with a similar strain, i.e., the same or similar strain of the species to which the bacterium belongs. In another embodiment, the activity of a protein may also be reduced as compared with *C. glutamicum* ATCC 13032. In another embodiment, the activity of a protein may also be reduced as compared with *C. glutamicum* ATCC 13869. In another embodiment, the activity of a protein may also be reduced as compared with *C. glutamicum* AJ12036 (FERM BP-734). In another embodiment, the activity of a protein may also be reduced as compared with *C. glutamicum* YDK010. The state that "the activity of a protein is reduced" also includes a state that the activity of the protein has completely disappeared. More specifically, the expression "the activity of a protein is reduced" may mean that the number of molecules of the protein per cell is reduced, and/or the function of each molecule of the protein is reduced as compared with those of an unmodified bacterium. That is, the term "activity" in the expression "the activity of a protein is reduced" is not limited to the catalytic activity of the protein, but may also mean the transcription amount of a gene (i.e., the amount of mRNA) encoding the protein or the translation amount of the gene (i.e., the amount of the protein). The phrase "the number of molecules of a protein per cell" may mean an average value per cell of the number of molecules of the protein. The state that "the number of molecules of the protein per cell is reduced" also includes a state that the protein does not exist at all. The state that "the function of each molecule of the protein is reduced" also includes a state that the function of each protein molecule has completely disappeared. The degree of the reduction in the activity of a protein is not particularly limited, so long as the activity is reduced as compared with that of an unmodified bacterium. The activity of a protein may be reduced to, for example, 50% or less, 20% or less, 10% or less, 5% or less, or 0% of that of an unmodified bacterium.

The modification for reducing the activity of a protein can be attained by, for example, reducing the expression of a gene encoding the protein. The expression "the expression of a gene is reduced" means that the expression of the gene is reduced as compared with an unmodified bacterium. Specifically, the expression "the expression of a gene is reduced" means that the expression amount of the gene per cell is reduced as compared with that of an unmodified bacterium. The phrase "the expression amount of a gene per cell" may mean an average value per cell of the expression amount of the gene. More specifically, the expression "the expression of a gene is reduced" may mean that the transcription amount of the gene (i.e., the amount of mRNA) is reduced, and/or the translation amount of the gene (i.e., the amount of the protein expressed from the gene) is reduced. The phrase that "the expression of a gene is reduced" also includes when the gene is not expressed at all. The phrase that "the expression of a gene is reduced" can also mean "the expression of a gene is attenuated". The expression of a gene may be reduced to, for example, 50% or less, 20% or less, 10% or less, 5% or less, or 0% of that of an unmodified bacterium.

The reduction in gene expression may be due to, for example, a reduction in the transcription efficiency, a reduction in the translation efficiency, or a combination of them. The expression of a gene can be reduced by modifying an expression control sequence of the gene. The term "expression control sequence" collectively refers to sites that affect the expression of a gene, such as a promoter, Shine-Dalgarno (SD) sequence (also referred to as ribosome-binding site (RBS)), and spacer region between RBS and the start codon. Expression control sequences can be identified by, for example, using a promoter search vector or gene analysis software such as GENETYX. When an expression control sequence is modified, one or more nucleotides, two or more nucleotides, three or more nucleotides of the expression control sequence are modified. The transcription efficiency of a gene can be reduced by, for example, replacing the promoter of the gene on a chromosome with a weaker promoter. The term "weaker promoter" means a promoter providing an attenuated transcription of a gene compared with an inherently existing wildtype promoter of the gene. Examples of weaker promoters include, for example, inducible promoters. That is, an inducible promoter may function as a weaker promoter under a non-induced condition, such as in the absence of the corresponding inducer. Furthermore, portion of or the entire expression control sequence may be deleted. The expression of a gene can also be reduced by, for example, manipulating a factor responsible for expression control. Examples of the factor responsible for expression control include low molecules responsible for transcription or translation control (inducers, inhibitors, etc.), proteins responsible for transcription or translation control (transcription factors, etc.), nucleic acids responsible for transcription or translation control (siRNA, etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described herein.

The modification for reducing the activity of a protein can also be attained by, for example, disrupting a gene encoding the protein. The expression "a gene is disrupted" means that a gene is modified so that a protein that can normally function is not produced. The phrase "a protein that normally functions is not produced" includes when the protein is not produced at all from the gene, and when the protein of which the function (such as activity or property) per molecule is reduced or eliminated is produced from the gene.

Disruption of a gene can be attained by, for example, deleting the gene on a chromosome. The term "deletion of a gene" refers to deletion of a partial or entire region of the coding region of the gene. Furthermore, the entire gene including sequences upstream and downstream from the coding region of the gene on a chromosome may be deleted. The sequences upstream and downstream from the coding region of the gene may include, for example, an expression control sequence of the gene. The region to be deleted may be any region such as an N-terminal region (region encoding an N-terminal region of a protein), an internal region, or a C-terminal region (region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing a mutation for an amino acid substitution (missense mutation), a stop codon (nonsense mutation), addition or deletion of one or two nucleotide residues (frame shift mutation), etc. into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the region to be deleted. The other nucleotide sequence is not particularly limited so long as a sequence that reduces or eliminates the activity of the encoded protein is chosen, and examples thereof include, for example, a marker gene such as antibiotic resistance genes, and a gene useful for production of an objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" refers to deletion of a partial or entire region of the amino acid sequence of the protein. In addition, the term "deletion of the amino acid sequence of a protein" means that the original amino acid sequence is not present in the protein, and also includes when the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a portion of or the entire coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be similarly applied to the position and length of the region to be deleted in deletion of the amino acid sequence of a protein.

In the case of Mdh protein, for example, at least 1 residue, 3 residues, 5 residues, 7 residues, 10 residues, 13 residues, 14 residues, 15 residues, 16 residues, 17 residues, 18 residues or 19 residues at the C-terminus of its amino acid sequence may be deleted. In the case of Mdh protein, in particular, at least 16 residues at the C-terminus of its amino acid sequence may be deleted. For example, in the case of the Mdh protein shown in SEQ ID NO 40, the amino acid sequence at positions 313-328 of SEQ ID NO 40 is the " 16 residues at the C-terminus". In the case of a Mdh protein, for example, at least the site corresponding to the above C-terminal site of SEQ ID NO 40 (e.g. the 16 residues at the C-terminus) in the amino acid sequence of the Mdh protein may be deleted. For the position of "the site corresponding to the above C-terminal site of SEQ ID NO 40" in any Mdh protein, the description of the position of the "amino acid residue at position X of the wildtype PhoS protein" described above can be applied.

Such modification of a gene on a chromosome as described above can be attained by, for example, preparing a disruption-type gene modified so that it is unable to produce a protein that normally functions, and transforming a host with a recombinant DNA containing the disruption-type gene to cause homologous recombination between the disruption-type gene and the wildtype gene on a chromosome and substitute the disruption-type gene for the wildtype gene on the chromosome. In this procedure, if a marker gene selected according to the characteristics of the host such as auxotrophy is included in the recombinant DNA, the operation becomes easier. Examples of the disruption-type gene include a gene in which a portion or the entire coding region is deleted, gene including a missense mutation, gene including a nonsense mutation, gene including a frame shift mutation, and gene inserted with an insertion sequence such as a transposon or marker gene. The protein encoded by the disruption-type gene has a conformation different from that of the wild-type protein, even if it is produced, and thus the function thereof is reduced or eliminated. The structure of the recombinant DNA to be used for homologous recombination is not particularly limited as long as it causes homologous recombination in a desired manner. For example, a host can be transformed with a linear DNA containing the disruption-type gene and further containing upstream and downstream sequences of the wildtype gene on the chromosome at the respective ends, so that homologous recombination occurs at each of upstream and downstream sides of the wildtype gene, thereby replacing the wildtype gene with the disruption-type gene in 1 action. The protein encoded by the disruption-type gene has a conformation different from that of the wild-type protein, even if it is produced, and thus the function thereof is reduced or eliminated. Such gene disruption based on gene substitution utilizing homologous recombination has already been established, and there are methods of using a linear DNA such as a method called "Red driven integration" (Datsenko, K. A, and Wanner, B. L., Proc. Natl. Acad. Sci. USA, 97:6640-6645 (2000)), and a method utilizing the Red driven integration in combination with an excision system derived from λ phage (Cho, E. H., Gumport, R I., Gardner, J. F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Pat. No. 6,303,383, Japanese Patent Laid-open (Kokai) No. 05-007491), and so forth. Such methods for modifying a chromosome using homologous recombination can be used for any modification on a chromosome, such as a modification of an expression control sequence, as well as for disruption of an objective gene.

A modification for reducing activity of a protein can also be attained by, for example, a mutagenesis treatment. Examples of the mutagenesis treatment include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

Such methods for reducing the activity of a protein as mentioned above may be used independently or in an arbitrary combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that of an unmodified bacterium. Examples of the method for evaluating the amount of mRNA include Northern hybridization, RT-PCR, microarray, RNA-Seq, and so forth (Sambrook, J., et al., Molecular Cloning: A Laboratory Manual/Third Edition, Cold Spring Harbor Laboratory Press, Cold Spring Harbor (USA), 2001). The amount of mRNA may be reduced to, for example, 50% or less, 20% or less, 10% or less, 5% or less, or 0% of that of an unmodified bacterium.

A reduction in the amount of a protein can be confirmed by performing SDS-PAGE and confirming the intensity of the separated protein band. A reduction in the amount of a protein can be confirmed by Western blotting using antibodies (Sambrook, J., et al., Molecular Cloning: A Laboratory Manual/Third Edition, Cold Spring Harbor Laboratory Press, Cold Spring Harbor (USA), 2001). The amount of the protein (such as the number of molecules of the protein per cell) may be reduced to, for example, 50% or less, 20% or less, 10% or less, 5% or less, or 0% of that of an unmodified bacterium.

Disruption of a gene can be confirmed by determining nucleotide sequence of a part or the whole of the gene, restriction enzyme map, full length, etc. of the gene depending on the means used for the disruption.

The aforementioned methods for reducing the activity of a protein as mentioned above can be applied to reduction in the activities of any proteins and reduction in the expression of any genes.

### <1-5> Methods for Increasing Expression of Gene

Hereinafter, methods for increasing the expression of a gene such as genes encoding the Tat secretion system will be explained.

The expression "the expression of a gene is increased" means that the expression of the gene is increased as compared with an unmodified bacterium. Specifically, the expression "the expression of a gene is increased" means that the expression amount of the gene per cell is increased as compared with that of an unmodified bacterium. The term "unmodified bacterium" refers to a control strain that has not been modified so that the activity of an objective protein is increased. Examples of the unmodified bacterium include a wildtype strain and parent strain. Specific examples of the unmodified bacterium include the respective type strains of the species of bacteria. Specific examples of the unmodified bacterium also include strains exemplified above in relation to the description of coryneform bacteria. That is, in an embodiment, the expression of a gene may be increased as compared with a type strain, i.e., the type strain of the species to which the chosen bacterium belongs. In another embodiment, the expression of a gene may also be increased as compared with *C. glutamicum* ATCC 13032. In another embodiment, the expression of a gene may also be increased as compared with *C. glutamicum* ATCC 13869. In another embodiment, the expression of a gene may also be increased as compared with C. *glutamicum* AJ12036 (FERM BP-734). In another embodiment, the expression of a gene may also be increased as compared with *C. glutamicum* YDK010. The term "the expression amount of a gene per cell" may mean an average value of the expression amount of the gene per cell. More specifically, the expression "the expression of a gene is increased" may mean that the transcription amount of the gene (i.e., the amount of mRNA) is increased, and/or the translation amount of the gene (i.e., the amount of the protein expressed from the gene) is increased. The state that "the expression of a gene is increased" may also be referred to as "the expression of a gene is enhanced". The degree of the increase in the expression of a gene is not particularly limited, so long as the expression of the gene is increased as compared with that of an unmodified bacterium. The expression of a gene may be increased to preferably 1.5 times or more, more preferably 2 times or more, or still more preferably 3 times or more of that of an unmodified bacterium. Furthermore, the expression "the expression of a gene is increased" can mean not only that the expression amount of an objective gene is increased in a strain that inherently expresses the objective gene, but also that the gene is introduced into a strain that does not inherently express the objective gene, and expressed therein. That is, the phrase "the expression of a gene is increased" may also include, for example, introduction of an objective gene into a strain that does not possess the gene, and expression of therein.

The expression of a gene can be increased by, for example, increasing the copy number of the gene.

The copy number of a gene can be increased by introducing the gene into the chromosome of a host. A gene can be introduced into a chromosome by, for example, using homologous recombination (MillerI, J. H., Experiments in Molecular Genetics, 1972, Cold Spring Harbor Laboratory). Examples of the gene transfer method utilizing homologous recombination include, for example, a method of using a linear DNA such as Red-driven integration (Datsenko, K. A., and Wanner, B. L., Proc. Natl. Acad. Sci. USA, 97:6640- 6645 (2000)), a method of using a plasmid containing a temperature sensitive replication origin, a method of using a plasmid capable of conjugative transfer, a method of using a suicide vector not having a replication origin that functions in a host, and a transduction method using a phage. Specifically, a host can be transformed with a recombinant DNA containing an objective gene, so that homologous recombination occurs at a target region on the chromosome of the host, thereby introducing the objective gene into the chromosome of the host. The structure of the recombinant DNA to be used for homologous recombination is not particularly limited as long as it causes homologous recombination in a desired manner. For example, a host can be transformed with a linear DNA containing an objective gene and further containing sequences of upstream and downstream of a target region on the chromosome at the respective ends of the objective gene, so that homologous recombination occurs at each of upstream and downstream of the target region, thereby replacing the target region with the objective gene. The recombinant DNA to be used for homologous recombination may contain a marker gene for selection of transformants. Only one copy, or two or more copies of a gene may be introduced. For example, by performing homologous recombination using a nucleotide sequence which is present in multiple copies on a chromosome as a target, multiple copies of a gene can be introduced into the chromosome. Examples of such a nucleotide sequence which is present in multiple copies on a chromosome include repetitive DNAs, and inverted repeats located at both ends of a transposon. Alternatively, homologous recombination may be performed by using an appropriate sequence on a chromosome such as a gene unnecessary for production of an objective substance as a target. Furthermore, a gene can also be randomly introduced into a chromosome by using a transposon or Mini-Mu (Japanese Patent Laid-open (Kokai) No. 2-109985, U.S. Pat. No. 5,882,888, EP805867B1). As the transposon, an artificial transposon may also be used (Japanese Patent Laid-open (Kokai) No. 9-70291). Such methods for modifying a chromosome using homologous recombination can be used for any modification on a chromosome, such as a modification of an expression control sequence, as well as for introduction of an objective gene.

Introduction of an objective gene into a chromosome can be confirmed by Southern hybridization using a probe having a sequence complementary to the whole gene or a part thereof, PCR using primers prepared on the basis of the sequence of the gene, etc.

Furthermore, the copy number of a gene can also be increased by introducing a vector containing the gene into a host. For example, the copy number of a target gene can be increased by linking a DNA fragment containing the target gene with a vector that functions in a host to construct an expression vector of the gene and transforming the host with the expression vector. The DNA fragment containing the target gene can be obtained by, for example, PCR using the genomic DNA of a microorganism having the target gene as the template. As the vector, a vector autonomously replicable in the cell of the host can be used. The vector can be a multi-copy vector. Furthermore, the vector can have a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, etc. Specific examples of vector autonomously replicable in coryneform bacteria include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 (Japanese Patent Laid-open (Kokai) No. 3-210184); plasmids pCRY21, pCRY2KE, pCRY2KX, pCRY31, pCRY3KE, and pCRY3KX (Japanese Patent Laid-open (Kokai) No. 2-72876 and U.S. Pat. No. 5,185,262); plasmids pCRY2 and pCRY3 (Japanese Patent Laid-open (Kokai) No. 1-191686); pAJ655, pAJ611, and pAJ1844 (Japanese Patent Laid-open (Kokai) No. 58-192900); pCG1 (Japanese Patent Laid-open (Kokai) No. 57-134500); pCG2 (Japanese Patent Laid-open (Kokai) No. 58-35197); pCG4 and pCG11 (Japanese Patent Laid-open (Kokai) No. 57-183799); pVK7 (Japanese Patent Laid-open (Kokai) No. 10-215883); pVK9 (US2006-0141588); pVC7 (Japanese Patent Laid-open (Kokai) No. 9-070291); pVS7 (WO2013/069634).

When a gene is introduced, it is sufficient that the gene is expressible by the host. Specifically, it is sufficient that the gene is harbored by a host so that it is expressed under control by a promoter that functions in the host. The promoter is not particularly limited so long as it functions in the host. The phrase "promoter that functions in a host" refers to a promoter that shows a promoter activity in the host. The promoter may be a promoter derived from the host, or a heterogenous promoter. The promoter may be the native promoter of the gene to be introduced, or a promoter of another gene. As the promoter, such a promoter as mentioned later which functions in a coryneform bacterium can be used.

A terminator for termination of gene transcription may be located downstream of the gene. The terminator is not particularly limited so long as it functions in the host. The terminator may be a terminator derived from the host, or a heterogenous terminator. The terminator may be the native terminator of the gene to be introduced, or a terminator of another gene.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more genes are introduced, it is sufficient that the genes each are expressible by the host. For example, all genes may be carried by a single expression vector or a chromosome. Furthermore, the genes may be separately carried by two or more expression vectors or separately carried by a single or two or more expression vectors and a chromosome. An operon having two or more genes may also be introduced.

The gene to be introduced is not particularly limited so long as it encodes a protein that functions in the host. The gene to be introduced may be a gene derived from or native to the host or may be a heterogenous gene. The gene to be introduced can be obtained by, for example, PCR using primers designed on the basis of the nucleotide sequence of the gene and using the genomic DNA of an organism having the gene, a plasmid carrying the gene, etc. as a template. The gene to be introduced may also be totally synthesized, for example, on the basis of the nucleotide sequence of the gene (Gene, 60(1), 115-127 (1987)). The obtained gene can be used as it is, or after being modified as required. That is, a gene can be modified to obtain a variant thereof. A gene can be modified by a known technique. For example, an objective mutation can be introduced into an objective site of DNA by the site-specific mutation method. That is, the coding region of a gene can be modified by the site-specific mutation method so that a specific site of the encoded protein includes substitution, deletion, insertion, and/or addition of amino acid residues. Examples of the site-specific mutation method include the method utilizing PCR (Higuchi, R., 61, in PCR Technology, Erlich, H. A. Eds., Stockton Press (1989); Carter, P, Meth. in Enzymol., 154, 382 (1987)), and the method utilizing phage (Kramer, W and Frits, H. J., Meth. in Enzymol., 154, 350 (1987); Kunkel, T. A. et al., Meth. in Enzymol., 154, 367 (1987)). Alternatively, a variant of a gene may be totally synthesized.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The phrase "expression control sequence" collectively refers to sites that affect the expression of a gene. Examples of the expression control sequence include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, homologous recombination. Examples of methods for modification using homologous recombination include a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" refers to a promoter providing improved transcription of a gene compared with an inherent wildtype promoter of the gene. Examples of stronger promoters usable in coryneform bacteria include the artificially modified P54-6 promoter (Appl. Microbiol. Biotechnol., 53, 674- 679 (2000)), pta, aceA, aceB, adh, and amyE promoters inducible in coryneform bacteria with acetic acid, ethanol, pyruvic acid, etc., cspB, SOD, and tuf (EF-Tu) promoters, which are other strong promoters capable of providing a large expression amount in coryneform bacteria (Journal of Biotechnology, 104 (2003) 311- 323; Appl. Environ. Microbiol., 2005 December; 71 (12):8587- 96), as well as lac promoter, tac promoter, and trc promoter. Furthermore, as the stronger promoter, a highly active existing promoter may also be obtained by using various reporter genes. For example, by making the -35 and -10 regions in a promoter region closer to the consensus sequence, the activity of the promoter can be enhanced (WO00/18935). Examples of highly active-type promoter include various tac-like promoters (Katashkina J I et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" means a SD sequence that provides an improved translation of mRNA compared with the inherent wildtype SD sequence of the gene. Examples of stronger SD sequences include, for example, RBS of the gene 10 derived from phage T7 (Olins P. O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon more frequently used. That is, the gene to be introduced may be modified, for example, so as to have optimal codons according to the codon usage frequency of a host to be used. Codons can be replaced by, for example, the site-specific mutation method. Alternatively, a gene fragment in which objective codons are replaced may be totally synthesized. Frequencies of codons in various organisms are disclosed in the "Codon Usage Database" (kazusa.or.jp/codon; Nakamura, Y. et al, Nucl. Acids Res., 28, 292 (2000)).

Furthermore, the expression of a gene can also be increased by amplifying a regulator that increases the expression of the gene or deleting or attenuating a regulator that reduces the expression of the gene.

Such methods for increasing the gene expression as mentioned above may be used independently or in any appropriate combination.

The method for the transformation is not particularly limited, and conventionally known methods can be used. There can be used, for example, a method of treating recipient cells with calcium chloride so as to increase the permeability thereof for DNA, which has been reported for the Escherichia coli K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C. H., Wilson, G. A. and Young, F. E., Gene, 1977, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S. N., 1979, Mol. Gen. Genet., 168:111- 115; Bibb, M. J., Ward, J. M. and Hopwood, O. A., 1978, Nature, 274:398- 400; Hinnen, A., Hicks, J. B. and Fink, G. R., 1978, Proc. Natl. Acad. Sci. USA, 75:1929- 1933). Transformation of coryneform bacteria can be carried out by, specifically, for example, the protoplast method (Gene, 39, 281-286(1985)), the electroporation method (Bio/Technology, 7, 1067-1070(1989)), the electric pulse method (Japanese Patent Laid-open (Kokai) No. 2-207791), or the like.

An increase in the expression of a gene can be confirmed by, for example, confirming an increase in the activity of the protein expressed from the gene. An increase in the activity of a protein can be confirmed by measuring the activity of the protein. For example, an increase in the activity of the Tat secretion system can be confirmed by confirming an increase in the secretory production amount of a protein fused with a Tat-dependent signal peptide at the N-terminus. In such a case, it is preferred that the secretory production amount of the protein fused with a Tat-dependent signal peptide at the N-terminus is increased to 1.5 times or more, 2 times or more, or 3 times or more, of that of an unmodified bacterium.

An increase in the expression of a gene can also be confirmed by, for example, confirming an increase in the transcription amount of the gene, or by confirming an increase in the amount of a protein expressed from the gene.

An increase of the transcription amount of a gene can be confirmed by comparing the amount of mRNA transcribed from the gene with that of an unmodified bacterium such as a wildtype strain or parent strain. Examples of the method for evaluating the amount of mRNA include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA (such as the number of molecules of the mRNA per cell) may be increased to, for example, 1.5 times or more, 2 times or more, or 3 times or more of that of an unmodified bacterium.

An increase in the amount of a protein can be confirmed by performing SDS-PAGE and confirming the intensity of the separated protein band. An increase in the amount of a protein can be confirmed by Western blotting using antibodies (Molecular Cloning, Cold Spring Harbor Laboratory Press, Cold Spring Harbor (USA), 2001). The amount of the protein (such as the number of molecules of the protein per cell) may be increased to, for example, 1.5 times or more, 2 times or more, or 3 times or more of that of an unmodified bacterium.

The aforementioned methods for increasing the expression of a gene can be used for enhancement of the expression of any genes.

### <1-6> Genetic construct for secretory expression of heterologous protein

It is known that a secretory protein is generally translated as a preprotein (also referred to as prepeptide) or a preproprotein (also referred to as prepropeptide), and then becomes a mature protein through processing. Specifically, a secretory protein is generally translated as a preprotein or preproprotein, then a signal peptide as the pre-moiety is cleaved with a protease (generally called signal peptidase), and the secretory protein is thereby converted into a mature protein or proprotein. As for the proprotein, the pro-moiety thereof is further cleaved by a protease, and the proprotein thereby becomes a mature protein. Therefore, a signal peptide is used for the secretory production of a heterologous protein in the method of the present invention. In the present invention, a preprotein and a preproprotein of a secretory protein may be collectively referred to as "secretory protein precursor". In the present invention, the "signal peptide" (also referred to as "signal sequence") refers to an amino acid sequence present at the N-terminus of a secretory protein precursor, and not usually present in the natural mature protein.

The genetic construct used for the present invention comprises, in the direction from 5' to 3', a promoter sequence that functions in a coryneform bacterium, a nucleic acid sequence encoding a signal peptide that functions in a coryneform bacterium, and a nucleic acid sequence encoding a heterologous protein. The nucleic acid sequence encoding the signal peptide may be ligated downstream from the promoter sequence so that the signal peptide is expressed under the control of the promoter. The nucleic acid sequence encoding the heterologous protein may be ligated downstream from the nucleic acid sequence encoding the signal peptide so that the heterologous protein is expressed as a fusion protein with the signal peptide. This fusion protein is also referred to as "fusion protein of the present invention". In the fusion protein of the present invention, the signal peptide and the heterologous protein may be or may not be adjacent to each other. That is, the expression "a heterologous protein is expressed as a fusion protein with a signal peptide" includes not only cases where a heterologous protein is expressed as a fusion protein with a signal peptide in which the signal peptide and the heterologous protein are adjacent to each other, but also include cases where a heterologous protein is expressed as a fusion protein in which the signal peptide and the heterologous protein are fused with each other via another amino acid sequence. For example, as described later, the fusion protein of the present invention can contain an insertion sequence, such as an amino acid sequence including Gln-Glu-Thr and an amino acid sequence used for enzymatic digestion, between the signal peptide and the heterologous protein. In addition, as described later, the heterologous protein eventually obtained may not have a signal peptide. That is, the expression "a heterologous protein is expressed as a fusion protein with a signal peptide" may simply mean that the heterologous protein constitutes a fusion protein with the signal peptide at the time of expression, and it may not necessarily mean that the eventually-obtained heterologous protein constitutes a fusion protein with the signal peptide. A promoter sequence is also referred to as "a promoter". A nucleic acid sequence may also be read as "gene". For example, a nucleic acid sequence encoding a heterologous protein is also referred to as "gene encoding a heterologous protein" or "heterologous protein gene". Examples of the nucleic acid sequence include DNA. The genetic construct used for the present invention may also include a control sequence (operator, SD sequence, terminator, etc.) effective for expression of the fusion protein of the present invention in a coryneform bacterium at such an appropriate position that it can function.

The promoter used in the present invention is not particularly limited so long as a promoter that functions in a coryneform bacterium is chosen. The promoter may be a promoter derived from a coryneform bacterium, such as one derived from the host, or it may be a heterologous promoter. The promoter may be the native promoter of the heterologous protein, or a promoter of another gene. The "promoter that functions in a coryneform bacterium" refers to a promoter that possesses promoter activity in a coryneform bacterium.

Specific examples of the heterologous promoter include, for example, promoters derived from *E. coli* such as *tac* promoter, *lac* promoter, *trp* promoter, and *araBAD* promoter. Among these, strong promoters such as *tac* promoter and inducible promoters such as *araBAD* promoter are preferred.

Examples of the promoter derived from a coryneform bacterium include, for example, promoters of the genes of the cell surface layer proteins PS1, PS2 (also referred to as CspB), and SlpA (also referred to as CspA), and promoters of various amino acid biosynthesis system genes. Specific examples of the promoters of various amino acid biosynthesis system genes include, for example, promoters of the glutamate dehydrogenase gene of the glutamic acid biosynthesis system, the glutamine synthetase gene of the glutamine synthesis system, the aspartokinase gene of the lysine biosynthesis system, the homoserine dehydrogenase gene of the threonine biosynthesis system, the acetohydroxy acid synthetase gene of the isoleucine and valine biosynthesis system, 2-isopropylmalate synthetase gene of the leucine biosynthesis system, the glutamate kinase gene of the proline and arginine biosynthesis system, the phosphoribosyl-ATP pyrophosphorylase gene of the histidine biosynthesis system, the deoxyarabinoheptulonate phosphate (DAHP) synthetase gene of the aromatic amino acid biosynthesis systems such as those for tryptophan, tyrosine, and phenylalanine, the phosphoribosyl pyrophosphate (PRPP) amidotransferase gene of the nucleic acid biosynthesis systems such as those for inosinic acid and guanylic acid, the inosinic acid dehydrogenase gene, and the guanylic acid synthetase gene.

Examples of the promoter that functions in a coryneform bacterium include such stronger promoters as described above usable in coryneform bacteria. As the promoter, a high activity type of an existing promoter may be obtained by using various reporter genes and used. For example, by making the - 35 and -10 regions in a promoter region closer to a consensus sequence, activity of the promoter can be enhanced (International Patent Publication WO00/18935). Examples of the method for evaluating strength of a promoter and strong promoters are described in the paper of Goldstein et al. (Prokaryotic promoters in biotechnology, Biotechnol. Annu. Rev., 1, 105-128 (1995)) and so forth. Furthermore, it is known that substitution, insertion, or deletion of several nucleotides in a spacer region between the ribosome-binding site (RBS) and the start codon, especially in a sequence immediately upstream of the start codon (5'-UTR), significantly affects stability and translation efficiency of mRNA, and these sequences can also be modified.

The signal peptide used in the present invention is not particularly limited so long as a signal peptide that functions in a coryneform bacterium is chosen. The signal peptide may be a signal peptide derived from a coryneform bacterium, such as one derived from the host, or it may be a heterologous signal peptide. The signal peptide may be the native signal peptide of the heterologous protein, or a signal peptide of another gene. The "signal peptide that functions in a coryneform bacterium" refers to a peptide that when it is ligated to the N-terminus of an objective protein, allows the coryneform bacterium to secrete the protein. Whether a signal peptide functions in a coryneform bacterium can be confirmed by, for example, expressing an objective protein in a form of being fused with the signal peptide, and confirming whether the protein is secreted.

Examples of the signal peptide include Tat-dependent signal peptides and Sec-dependent signal peptides.

The term "Tat-dependent signal peptide" refers to a signal peptide recognized by the Tat system. The term "Tat-dependent signal peptide" may specifically refer to a signal peptide that, upon being linked at the N-terminus of an objective protein, results in secretion of the protein by the Tat secretion system.

Examples of the Tat-dependent signal peptide include the signal peptide of the TorA protein (trimethylamine-N-oxidoreductase) of *E. coli,* the signal peptide of SufI protein (suppressor of ftsI) of *E. coli,* the PhoD protein (phosphodiesterase) of *Bacillus subtilis,* the signal peptide of LipA protein (lipoic acid synthase) of *Bacillus subtilis,* and the signal peptide of IMD protein (isomaltodextranase) of *Arthrobacter globiformis.* The amino acid sequences of these signal peptides are as follows.
TorA signal peptide:
   MNNNDLFQASRRRFLAQLGGLTVAGMLGPSLLTPRRATA (SEQ ID NO: 75)
SufI signal peptide: MSLSRRQFIQASGIALCAGAVPLKASA (SEQ ID NO: 76)
PhoD signal peptide:
   MAYDSRFDEWVQKLKEESFQNNTFDRRKFIQGAGKIAGLSLGLTIAQS (SEQ ID NO: 77)
LipA signal peptide: MKFVKRRTTALVTTLMLSVTSLFALQPSAKAAEH (SEQ ID NO: 78)
IMD signal peptide: MMNLSRRTLLTTGSAATLAYALGMAGSAQA (SEQ ID NO: 79)

The Tat-dependent signal peptide has a twin-arginine motif. Examples of the twin-arginine motif include S/T-R-R-X-F-L-K (SEQ ID NO: 23) and R-R-X-#-# (X: naturally occurring amino acid residue; #: hydrophobic amino acid residue).

The term "Sec-dependent signal peptide" refers to a signal peptide recognized by the Sec system. The term "Sec-dependent signal peptide" may specifically refer to a signal peptide that, upon being linked at the N-terminus of an objective protein, results in secretion of the protein by the Sec secretion system.

Examples of the Sec-dependent signal peptide include a signal peptide of a cell surface layer protein of a coryneform bacterium. The cell surface layer protein of coryneform bacteria is as described above. Examples of the cell surface layer protein of coryneform bacteria include PS1 and PS2 (CspB) derived from C. *glutamicum* (Japanese Patent Laid-open (Kohyo) No. 6-502548), and SlpA (CspA) derived from C. *ammoniagenes (C. stationis)* (Japanese Patent Laid-open (Kokai) No. 10-108675). The amino acid sequence of the signal peptide of PS1 (PS1 signal peptide) of C. *glutamicum* is shown in SEQ ID NO: 25, the amino acid sequence of the signal peptide of PS2 (CspB) (PS2 signal peptide) of C. *glutamicum* is shown in SEQ ID NO: 83, and the amino acid sequence of the signal peptide of SlpA (CspA) (SlpA signal peptide)of C. stationis is shown in SEQ ID NO: 27.

The Tat-dependent signal peptide may be a variant of any of the Tat-dependent signal peptides exemplified above, so long as it contains a twin-arginine motif and the original function thereof is maintained. The Sec-dependent signal peptide may be a variant of any of the Sec-dependent signal peptides exemplified above, so long as the original function thereof is maintained. The above descriptions concerning conservative variants of the mdh gene and Mdh protein can be similarly applied to variants of the signal peptide and the gene encoding it. For example, the signal peptide may be a peptide having any of the aforementioned amino acid sequences, but including substitution, deletion, insertion, and/or addition of one or several amino acid residues at one or several positions. The number meant by the term "one or several" used for a variant of the signal peptide is specifically, preferably 1 to 7, more preferably 1 to 5, still more preferably 1 to 3, particularly preferably 1 to 2. In the present invention, the terms "TorA signal peptide", "SufI signal peptide", "PhoD signal peptide", "LipA signal peptide", "IMD signal peptide", "PS1 signal peptide", "PS2 signal peptide", and "SlpA signal peptide" include not only the peptides of SEQ ID NOS: 18-22, and 25-27, respectively, but also includes conservative variants thereof.

The expression "original function is maintained" used for the Tat-dependent signal peptide means that the peptide is recognized by the Tat system, and specifically, may mean that the peptide has a function of, upon being linked at the N-terminus of an objective protein, resulting in secretion of the protein by the Tat secretion system. Whether a peptide function as the Tat-dependent signal peptide can be confirmed by, for example, confirming an increase in the secretory production amount of a protein linked with the peptide at the N-terminus due to enhancement of the Tat secretion system, or confirming a reduction in the secretory production amount of a protein linked with the peptide at the N-terminus due to deletion of the Tat secretion system.

The expression "original function is maintained" used for the Sec-dependent signal peptide means that the peptide is recognized by the Sec system, and specifically, may mean that the peptide has a function of, upon being linked at the N-terminus of an objective protein, resulting in secretion of the protein by the Sec secretion system. Whether a peptide function as the Sec-dependent signal peptide can be confirmed by, for example, confirming an increase in the secretory production amount of a protein linked with the peptide at the N-terminus due to enhancement of the Sec secretion system, or confirming a reduction in the secretory production amount of a protein linked with the peptide at the N-terminus due to deletion of the Sec secretion system.

The signal peptide is generally cleaved by a signal peptidase, when the translation product is secreted out of the cell. That is, the heterologous protein eventually obtained may not have a signal peptide. As a gene encoding a signal peptide, although a naturally occurring gene may be used as it is, it may be modified so that it has the optimal codons according to codon frequencies in a host to be used.

In the genetic construct used for the present invention, a nucleic acid sequence encoding an amino acid sequence including Gln-Glu-Thr may be inserted between the nucleic acid sequence encoding the signal peptide and the nucleic acid sequence encoding the heterologous protein (WO2013/062029). The "amino acid sequence including Gln-Glu-Thr" is also referred to as "insertion sequence used for the present invention". Examples of the insertion sequence used for the present invention include amino acid sequences including Gln-Glu-Thr described in WO2013/062029. Particularly, the insertion sequence used for the present invention can be used preferably in combination with the Sec-dependent signal peptide.

The insertion sequence used for the present invention is preferably a sequence having 3 or more amino acid residues from the N-terminus of the mature protein of the cell surface layer protein CspB of a coryneform bacterium (henceforth also referred to as "mature CspB" or "CspB mature protein"). The term "sequence having 3 or more amino acid residues from the N-terminus" refers to an amino acid sequence starting from the amino acid residue at position 1 of the N-terminus to an amino acid residue at position 3 or a more remote position.

The cell surface layer protein CspB of coryneform bacteria is as described above. Specific examples of CspB include, for example, CspB of C. *glutamicum* ATCC 13869, CspB of 28 strains of C .*glutamicum* exemplified above, and variants thereof. In the amino acid sequence of the CspB protein of *C. glutamicum* ATCC 13869 shown in SEQ ID NO: 11, the amino acid residues at positions 1 to 30 correspond to the signal peptide, and the amino acid residues at positions 31 to 499 correspond to the CspB mature protein. The amino acid sequence of the CspB mature protein of C. *glutamicum* ATCC 13869 except for the 30 amino acid residues as the signal peptide moiety is shown in SEQ ID NO: 28. In the mature CspB of C. *glutamicum* ATCC 13869, the amino acid residues at positions 1 to 3 of the N-terminus correspond to Gln-Glu-Thr.

The insertion sequence used for the present invention is preferably an amino acid sequence starting from the amino acid residue at position 1 to an amino acid residue at any of the positions 3 to 50 of the mature CspB. The insertion sequence used for the present invention is more preferably an amino acid sequence starting from the amino acid residue at position 1 to an amino acid residue at any of the positions 3 to 8, 17, and 50 of the mature CspB. The insertion sequence used for the present invention is particularly preferably an amino acid sequence starting from the amino acid residue at position 1 to an amino acid residue at any of the positions 4, 6, 17 and 50.

The insertion sequence used for the present invention is preferably an amino acid sequence selected from the group consisting of the following amino acid sequences (A) to (H):
(A) Gln-Glu-Thr
(B) Gln-Glu-Thr-Xaa1
(C) Gln-Glu-Thr-Xaa1-Xaa2
(D) Gln-Glu-Thr-Xaa1-Xaa2-Xaa3
(E) an amino acid sequence having Gln-Glu-Thr fused with the amino acid residues at positions 4 to 7 of a mature CspB,
(F) an amino acid sequence having Gln-Glu-Thr fused with the amino acid residues at positions 4 to 8 of a mature CspB,
(G) an amino acid sequence having Gln-Glu-Thr fused with the amino acid residues at positions 4 to 17 of a mature CspB,
(H) an amino acid sequence having Gln-Glu-Thr fused with the amino acid residues at positions 4 to 50 of a mature CspB.

In the amino acid sequences (A) to (H), Xaa1 is Asn, Gly, Thr, Pro, or Ala, Xaa2 is Pro, Thr, or Val, and Xaa3 is Thr or Tyr. As for the amino acid sequences (A) to (H), "Gln-Glu-Thr fused with the amino acid residues at positions 4 to X of a mature CspB" means that the amino acid residues at positions 4 to X of the N-terminus of a mature CspB is fused to Thr of Gln-Glu-Thr. The first to third amino acid residues of the N-terminus of a mature CspB are usually Gln-Glu-Thr, and in such a case, "an amino acid sequence having Gln-Glu-Thr fused with the amino acid residues at positions 4 to X of a mature CspB" is synonymous with an amino acid sequence having the amino acid residues at position 1 to X of the mature CspB.

Furthermore, specifically, the insertion sequence used for the present invention is preferably an amino acid sequence selected from the group consisting of Gln-Glu-Thr-Asn-Pro-Thr (SEQ ID NO: 32), Gln-Glu-Thr-Gly-Thr-Tyr (SEQ ID NO: 33), Gln-Glu-Thr-Thr-Val-Thr (SEQ ID NO: 34), Gln-Glu-Thr-Pro-Val-Thr (SEQ ID NO: 35), and Gln-Glu-Thr-Ala-Val-Thr (SEQ ID NO: 36).

In the present invention, the "amino acid residue at position X of the mature CspB" refers to an amino acid residue corresponding to the amino acid residue at position X in SEQ ID NO: 28. Which amino acid residue is the "amino acid residue corresponding to the amino acid residue at position X in SEQ ID NO: 28" in the amino acid sequence of an arbitrary mature CspB can be determined by alignment between the amino acid sequence of the arbitrary mature CspB and the amino acid sequence of SEQ ID NO: 28.

Examples of the heterologous protein to be secretory produced according to the method of the present invention include, for example, physiologically active proteins, receptor proteins, antigenic proteins to be used as vaccines, enzymes, and any other protein.

Examples of the enzymes include, for example, transglutaminase, protein glutaminase, isomaltodextranase, protease, endopeptidase, exopeptidase, aminopeptidase, carboxypeptidase, collagenase, chitinase, and so forth. Examples of transglutaminase include, for example, secretory-type transglutaminases of Actinomycetes such as *Streptoverticillium mobaraense* IFO 13819 (WO01/23591), *Streptoverticillium cinnamoneum* IFO 12852, *Streptoverticillium griseocarneum* IFO 12776, and *Streptomyces lydicus* (WO96/06931), and of filamentous fungi such as Oomycetes (WO96/22366). Examples of protein glutaminase include, for example, protein glutaminase of *Chryseobacterium proteolyticum* (WO2005/103278). Examples of isomaltodextranase include, for example, isomaltodextranase of *Arthrobacter globiformis* (WO2005/103278).

Examples of the physiologically active proteins include, for example, growth factors, hormones, cytokines, and antibody-related molecules.

Specific examples of the growth factors include, for example, Epidermal growth factor (EGF), Insulin-like growth factor-1 (IGF-1), Transforming growth factor (TGF), Nerve growth factor (NGF), Brain-derived neurotrophic factor (BDNF), Vascular endothelial growth factor (VEGF), Granulocyte-colony stimulating factor (G-CSF), Granulocyte-macrophage-colony stimulating factor (GM-CSF), Platelet-derived growth factor (PDGF), Erythropoietin (EPO), Thrombopoietin (TPO), acidic fibroblast growth factor (aFGF or FGF1), basic fibroblast growth factor (bFGF or FGF2), keratinocyte growth factor (KGF-1 or FGF7, and, KGF-2 or FGF10), and Hepatocyte growth factor (HGF).

Specific examples of the hormones include, for example, insulin, glucagon, somatostatin, human growth hormone (hGH), parathyroid hormone (PTH), calcitonin, and exenatide.

Specific examples of the cytokines include, for example, interleukins, interferons, and tumor necrosis factors (TNFs).

The growth factors, hormones, and cytokines may not be strictly distinguished from one another. For example, a physiologically active protein may be a protein belonging to a single group selected from growth factors, hormones, and cytokines, or may be a protein belonging to a plurality of groups selected from those.

Furthermore, a physiologically active protein may be an intact protein, or may be a part of a protein. Examples of a part of a protein include, for example, a part having physiological activity. Specific examples of a part having physiological activity include, for example, Teriparatide, a physiologically active peptide consisting of the N-terminal 34 amino acid residues of parathyroid hormone (PTH).

The term "antibody-related molecule" refers to a protein containing a molecular species having a single domain or a combination of two or more domains selected from the domains constituting a complete antibody. Examples of the domains constituting a complete antibody include heavy chain domains VH, CH1, CH2, and CH3, and light chain domains VL and CL. The antibody-related molecule may be a monomeric protein, or may be a multimeric protein, so long as it contains the above-mentioned molecular species. When the antibody-related molecule is a multimeric protein, it may be a homo-multimer having a single kind of subunit, or may be a hetero-multimer having two or more kinds of subunits. Specific examples of the antibody-related molecules include, for example, complete antibody, Fab, F(ab'), F(ab')₂, Fc, dimer having a heavy chain (H chain) and a light chain (L chain), Fc-fusion protein, heavy chain (H chain), light chain (L chain), light chain Fv (scFv), sc(Fv)₂, disulfide-bonded Fv (sdFv), diabody and VHH fragment (Nanobody(registered trademark)). More specific examples of the antibody-related molecules include, for example, Trastuzumab, Adalimumab, and Nivolumab.

The receptor proteins are not particularly limited. A receptor protein may be, for example, a receptor protein for any of physiologically active proteins and other physiologically active substances. Examples of the other physiologically active substances include, for example, neurotransmitters such as dopamine. Furthermore, a receptor protein may be an orphan receptor of which the corresponding ligand is not known.

The antigen proteins to be used as vaccines are not particularly limited, so long as they are proteins that can induce an immune response. An antigen protein can be appropriately selected depending on the intended object of the immune response.

In addition, examples of other proteins include Liver-type fatty acid-binding protein (LFABP), fluorescent protein, immunoglobulin-binding protein, albumin, and extracellular protein. Examples of the fluorescent protein include green fluorescent protein (GFP). Examples of the immunoglobulin-binding protein include Protein A, Protein G, and Protein L. Examples of albumin include human serum albumin.

Examples of the extracellular protein include fibronectin, vitronectin, collagen, osteopontin, laminin, and partial sequences thereof. Laminin is a protein having a heterotrimeric structure having an α chain, a β chain, and a γ chain. Examples of laminin include laminin of mammals. Examples of the mammals include primates such as human, monkey, and chimpanzee; rodents such as mouse, rat, hamster, and guinea pig; and other various mammals such as rabbit, horse, cattle, sheep, goat, pig, dog, and cat. Particular examples of the mammals include human. Examples of the subunit chains of laminin (i.e., a, (α, β, and γ chains) include 5 kinds of α chains (α1 to α5), 3 kinds of β chains (β1 to β3), and 3 kinds of γ chains (γ1 to γ3). Laminin constitutes various isoforms depending on combinations of these subunits. Specific examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. Examples of the partial sequence of laminin include laminin E8, which is an E8 fragment of laminin. Laminin E8 is a protein having a heterotrimeric structure having an E8 fragment of α chain (α chain E8), an E8 fragment of β chain (β chain E8), and an E8 fragment of γ chain (γ chain E8). The subunit chains of laminin E8 (i.e., α chain E8, β chain E8, and γ chain E8) are also collectively referred to as "E8 subunit chains". Examples of the E8 subunit chains includes E8 fragments of the laminin subunit chains exemplified above. Laminin E8 constitutes various isoforms depending on combinations of these E8 subunit chains. Specific examples of laminin E8 include, for example, laminin 111E8, laminin 121E8, laminin 211E8, laminin 221E8, laminin 332E8, laminin 421E8, laminin 411E8, laminin 511E8, and laminin 521E8.

A gene encoding a heterologous protein, such as these proteins, can be used as it is, or after being modified as required. A gene encoding a heterologous protein can be modified according to a host to be used and/or for obtaining a desired activity. For example, the gene encoding a heterologous protein may be modified so that the encoded heterologous protein include addition, deletion, substitution, or the like of one or several amino acid residue(s). The above descriptions concerning variants of the Mdh protein and mdh gene can be similarly applied to the heterologous protein to be secretory produced by the method of the present invention and the gene encoding it. A protein specified with the type of organism from which the protein is derived is not limited to proteins per se found in that organism and shall also include proteins having any of the amino acid sequences of proteins found in that organism and variants thereof. These variants may or may not be found in the organism. That is, for example, the term "protein derived from human" is not limited to proteins per se found in human and shall also include proteins having any of the amino acid sequences of proteins found in human and variants thereof. Furthermore, in the gene encoding a heterologous protein, an arbitrary codon may be replaced with an equivalent codon thereof. For example, in the gene encoding a heterologous protein may be modified so as to have optimal codons according to the codon usage frequency of the chosen host.

The genetic construct of the present invention may further include a nucleic acid sequence encoding an amino acid sequence used for enzymatic digestion between the nucleic acid sequence encoding the amino acid sequence including Gln-Glu-Thr and the nucleic acid sequence encoding the heterologous protein. If the amino acid sequence used for enzymatic digestion is inserted in the fusion protein of the present invention, the expressed fusion protein can be enzymatically digested to obtain the objective heterologous protein.

The amino acid sequence used for enzymatic digestion is not particularly limited so long as it is a sequence that can be recognized and digested by an enzyme that hydrolyzes a peptide bond, and a usable sequence can be appropriately chosen according to the amino acid sequence of the objective heterologous protein. The nucleic acid sequence encoding the amino acid sequence used for enzymatic digestion may be designed on the basis of that amino acid sequence. The nucleic acid sequence encoding the amino acid sequence used for enzymatic digestion may be designed, for example, optimal codons can be used according to codon frequencies observed in the host.

The amino acid sequence used for enzymatic digestion is preferably a recognition sequence of a protease showing high substrate specificity. Specific examples of such an amino acid sequence include, for example, a recognition sequence of factor Xa protease and a recognition sequence of proTEV protease. The factor Xa protease and the proTEV protease recognize the amino acid sequence of Ile-Glu-Gly-Arg (= IEGR, SEQ **ID** NO: 37) and the amino acid sequence of Glu-Asn-Leu-Tyr-Phe-Gln (= ENLYFQ, SEQ ID NO: 38) in a protein, respectively, to specifically digest the protein at the C-terminal side of each recognition sequence.

The N-terminal region of the heterologous protein eventually obtained by the method of the present invention may be the same as that of the natural protein, or may not be the same as that of the natural protein. For example, the N-terminal region of the eventually obtained heterologous protein may be that of the natural protein including addition or deletion of one or several amino acid residues. Although the number of the "one or several" amino acid residues may differ depending on the full length or structure of the objective heterologous protein, specifically, it is preferably 1 to 20, more preferably 1 to 10, still more preferably 1 to 5, particularly preferably 1 to 3.

Furthermore, the heterologous protein to be secretory produced may be a protein that includes a pro-structure moiety (proprotein). When the heterologous protein to be secretory produced is a proprotein, the heterologous protein to be eventually obtained may be the proprotein or may not be the proprotein. That is, the proprotein may be processed into the mature protein by cleavage of the pro-structure moiety. The cleavage can be attained with, for example, a protease. When a protease is used, generally, the proprotein is preferably cleaved at a position substantially the same as that of the natural protein, or more preferably at exactly the same position as that of the natural protein so that the same mature protein as the natural mature protein is obtained, in view of the activity of the eventually obtained protein. Therefore, generally, a specific protease that cleaves the proprotein at such a position that the same protein as the naturally occurring mature protein is generated is the most desirable. However, the N-terminal region of the heterologous protein to be eventually obtained may not be the same as that of the natural protein as described above. For example, depending on type, purpose of use etc. of the heterologous protein to be produced, a protein having an N-terminus longer or shorter by one to several amino acid residues compared with the natural protein may have more appropriate activity. Proteases usable in the present invention include, for example, commercially available proteases such as Dispase (produced by Boehringer Mannheim) as well as those obtainable from culture broth of a microorganism such as culture broth of actinomycetes. Such proteases may be used in an un-purified state or may be used after purification to an appropriate purity as required. When the pro-structure moiety is cleaved to obtain a mature protein, the inserted amino acid sequence including Gln-Glu-Thr is removed together with the pro-structure moiety, and therefore the objective protein can be obtained without providing an amino acid sequence used for enzymatic digestion downstream from the amino acid sequence including Gln-Glu-Thr.

The method for introducing the genetic construct used for the present invention into the coryneform bacterium is not particularly limited. The term "introduction of the genetic construct used for the present invention" refers to making a host harbor the genetic construct. The term "introduction of the genetic construct used for the present invention" includes not only cases where the genetic construct that has been preliminarily constructed is collectively introduced into a host, but also includes cases where at least the heterologous protein gene is introduced into a host and the genetic construct is constructed in the host. In the bacterium of the present invention, the genetic construct used for the present invention may be present on a vector that autonomously replicates out of the chromosome such as a plasmid, or may be incorporated into the chromosome. The genetic construct used for the present invention can be introduced, for example, in the same manner as that for introduction of a gene in methods for increasing the expression of a gene described above. In addition, for constructing the bacterium of the present invention, introduction of the genetic structure used for the present invention, reduction of the activity of Mdh protein, and other modifications can be performed in an arbitrary order.

The genetic construct used for the present invention can be introduced into a host by using, for example, a vector that includes the genetic construct. For example, the genetic construct used for the present invention can be introduced into a host by ligating the genetic construct with a vector to construct an expression vector of the genetic construct, and transforming the host with the expression vector. Also, when the vector contains a promoter that functions in a coryneform bacterium, an expression vector of the genetic construct used for the present invention can be constructed by ligating the nucleic acid sequence encoding the fusion protein of the present invention downstream from the promoter. The vector is not particularly limited so long as a vector autonomously replicable in a coryneform bacterium is chosen. The vector usable in a coryneform bacterium is as described above.

Furthermore, the genetic construct used for the present invention can be introduced into the chromosome of a host by using, for example, a transposon such as an artificial transposon. When a transposon is used, the genetic construct used for the present invention is introduced into the chromosome by homologous recombination or translocation ability of the transposon itself. Furthermore, the genetic construct used for the present invention can also be introduced into the chromosome of a host by other introduction methods utilizing homologous recombination. Examples of the introduction methods utilizing homologous recombination include, for example, methods utilizing a linear DNA, a plasmid having a temperature sensitive replication origin, a plasmid capable of conjugative transfer, a suicide vector not having a replication origin that functions in a host, and so forth. **In** addition, at least the heterologous protein gene may be introduced into the chromosome so that the genetic construct used for the present invention is constituted on the chromosome. **In** this case, a part or all of the constituents contained in the genetic construct, other than the heterologous protein gene, may be inherently present on the chromosome of the host. Specifically, for example, by using a promoter sequence inherently present on the chromosome of the host and a nucleic acid sequence encoding a signal peptide inherently present on the chromosome of the host and ligated downstream from the promoter sequence as they are, and replacing only the gene ligated downstream from the nucleic acid sequence encoding the signal peptide with an objective heterologous protein gene, the genetic construct used for the present invention can be constituted on the chromosome, and the bacterium of the present invention can be thereby constructed. A part of the genetic construct used for the present invention, such as the heterologous protein gene, can be introduced into the chromosome in the same manner as that for introduction of the genetic construct used for the present invention into the chromosome.

The genetic construct used for the present invention or a constituent thereof, such as promoter sequence, nucleic acid sequence encoding a signal peptide, or nucleic acid sequence encoding a heterologous protein, can be obtained by, for example, cloning. Specifically, for example, the genetic construct used for the present invention can be obtained by obtaining an objective heterologous protein gene by cloning from an organism having the objective heterologous protein, and then subjecting the gene to modification such as introduction of the nucleic acid sequence encoding the signal peptide and introduction of the promoter sequence. Furthermore, the genetic construct used for the present invention or a constituent thereof can also be obtained by chemical synthesis (Gene, 60(1), 115-127 (1987)). The obtained genetic construct used for the present invention or constituent thereof can be used as it is, or after being modified as required.

Furthermore, when two or more kinds of proteins are expressed, it is sufficient that the genetic constructs for secretory expression of the proteins are harbored by the bacterium of the present invention so that secretory expression of the objective heterologous proteins can be attained. Specifically, for example, all the genetic constructs for secretory expression of the proteins may be harbored on a single expression vector, or harbored on the chromosome. Alternatively, the genetic constructs for secretory expression of the proteins may be separately harbored on a plurality of expression vectors, or may be separately harbored on one or more expression vectors and the chromosome. The "case where two or more kinds of proteins are expressed" refers to, for example, a case where two or more kinds of heterologous proteins are secretory produced, or a case where a hetero-multimeric protein is secretory produced.

The method for introducing the genetic construct used for the present invention into the coryneform bacterium is not particularly limited, and a generally used method, for example, the protoplast method (Gene, 39, 281-286 (1985)), the electroporation method (Bio/Technology, 7, 1067-1070 (1989)), the electric pulse method (Japanese Patent Laid-open (Kokai) No. 2-207791), and so forth can be used.

### <2> Method for producing heterologous protein

By culturing the bacterium of the present invention obtained as described above to express a heterologous protein, a large amount of the heterologous protein secreted out of the cells is obtained.

The bacterium of the present invention can be cultured according to a usually used method and conditions. For example, the bacterium of the present invention can be cultured in a usual medium containing a carbon source, a nitrogen source, and inorganic ions. In order to obtain still higher proliferation, organic micronutrients such as vitamins and amino acids can also be added as required.

As the carbon source, carbohydrates such as glucose and sucrose, organic acids such as acetic acid, alcohols, and others can be used. As the nitrogen source, ammonia gas, aqueous ammonia, ammonium salts, and others can be used. As the inorganic ions, calcium ions, magnesium ions, phosphate ions, potassium ions, iron ions, and so forth are appropriately used as required. The culture is performed within appropriate ranges of pH 5.0 to 8.5 and 15 to 37°C under aerobic conditions for 1 to 7 days. Furthermore, the culture conditions for L-amino acid production by coryneform bacteria and other conditions described for the methods for secretory producing a protein using a Sec- or Tat-dependent signal peptide can be used (refer to WO01/23591 and WO2005/103278). Furthermore, when an inducible promoter is used for expression of the heterologous protein, culture may also be performed with adding a promoter-inducing agent to the medium. By culturing the bacterium of the present invention under such conditions, a large amount of the objective protein is produced in cells and efficiently secreted out of the cells. In addition, according to the method of the present invention, the produced heterologous protein is secreted out of the cells, and therefore a protein that is generally lethal if it is accumulated in a large amount in cells of microorganisms, such as transglutaminases, can also be continuously produced without lethal effect.

The protein secreted in the medium according to the method of the present invention can be separated and purified from the medium after the culture by a method well known to those skilled in the art. For example, after the cells are removed by centrifugation or the like, the protein can be separated and purified by a known appropriate method such as salting out, ethanol precipitation, ultrafiltration, gel filtration chromatography, ion exchange column chromatography, affinity chromatography, medium or high pressure liquid chromatography, reverse phase chromatography, and hydrophobic chromatography, or a combination of these. Furthermore, in a certain case, culture or culture supernatant may be used as it is. The protein secreted in the cell surface layer according to the method of the present invention can also be separated and purified in the same manner as that for the case where the protein is secreted in the medium, after solubilizing it by a method well known to those skilled in the art such as elevation of salt concentration and use of a surfactant. Furthermore, in a certain case, the protein secreted in the cell surface layer may be used as, for example, an immobilized enzyme, without solubilizing it.

Secretory production of the objective heterologous protein can be confirmed by performing SDS-PAGE for the culture supernatant and/or a fraction containing the cell surface layer as a sample, and confirming the molecular weight of the separated protein band. Furthermore, secretory production of the objective heterologous protein can also be confirmed by performing Western blotting using antibodies for the culture supernatant and/or a fraction containing the cell surface layer as a sample (Molecular Cloning, Cold Spring Harbor Laboratory Press, Cold Spring Harbor (USA), 2001). Furthermore, secretory production of the objective heterologous protein can also be confirmed by detecting an N-terminal amino acid sequence of the objective protein using a protein sequencer. Furthermore, secretory production of the objective heterologous protein can also be confirmed by determining the mass of the objective protein using a mass spectrometer. Furthermore, when the objective heterologous protein is an enzyme or a protein having a certain measurable physiological activity, secretory production of the objective heterologous protein can be confirmed by measuring enzymatic activity or the physiological activity of the objective protein in the culture supernatant and/or a fraction containing the cell surface layer as a sample.

### Examples

The invention is described in more detail below with reference to nonlimiting examples.

Example 1: Construction of Corynebacterium glutamicum lacking the malate dehydrogenase gene mdh.

### (1) Construction of vector pBS5TΔ2278 for mdh gene deletion.

The genome sequence of strain C. glutamicum ATCC13869 and the nucleotide sequence of the mdh gene encoding malate dehydrogenase (hereafter referred to as Mdh) have already been determined (Genbank Accession No. AP017557, NCBI locus_tag CGBL_0122780). The nucleotide sequence of the mdh gene is shown in SEQ ID NO 39 and the amino acid sequence of Mdh is shown in SEQ ID NO 40.

PCR was performed using chromosomal DNA of C. glutamicum ATCC13869 strain prepared with the PurElute^{™} Genomic DNA Kit (EdgeBio) as a template, in combination with primers SEQ ID NO 41 and SEQ ID NO 42 to amplify a region of approximately 1 kbp upstream of the mdh gene, and in combination with primers SEQ ID NO 43 and SEQ ID NO 44 to amplify a region of approximately 1 kbp downstream of the 3' side of the mdh gene, respectively. Pyrobest(R) DNA polymerase (Takara Bio) was used for PCR and the reaction conditions followed the protocol recommended by the supplier. After agarose gel electrophoresis of the each amplified DNA fragment of approximately 1 kbp, the bands of interest were cut out and collected from the gel using the Wizard(R) SV Gel and PCR Clean-Up System (Promega). The collected two DNA fragments were inserted into the SmaI site of pBS5T as described in WO2006/057450 by an infusion reaction to obtain the vector pBS5TΔ2278 for mdh gene deletion. The In-Fusion(R) HD Cloning Kit (Takara Bio) was used for the infusion reaction and the reaction conditions followed the protocol recommended by the supplier.

### (2) Construction of a YDK010::phoS(W302C) strain deficient in the mdh gene.

The YDK010::phoS(W302C) strain described in WO2016/171224 was transformed with pBS5TΔ2278 constructed in Example 1(1). From the resulting transformant, a strain was selected according to the method described in WO2006/057450 to obtain the YDK010::phoS(W302C)Δ2278 strain lacking the mdh gene.

Example 2: Construction of Corynebacterium glutamicum with a stop codon inserted in the malate dehydrogenase gene mdh.

### (1) Construction of vector pBS5Ts2278 for insertion of a stop codon into the mdh gene.

The following method was used to obtain a strain in which the mdh gene was modified to encode a C-terminal deleted Mdh by inserting a stop codon into the mdh gene, thereby deleting the amino acid sequence after position 313 of Mdh, which has 328 residues.

PCR was performed using chromosomal DNA of C. glutamicum ATCC13869 strain prepared with the PurElute^{™} Genomic DNA Kit (EdgeBio) as a template, in combination with primers SEQ ID NO 45 and SEQ ID NO 46 to amplify a region of approximately 1 kbp 5' upstream of GCG encoding Ala at position 312 of Mdh, and a region of approximately 1 kbp 3' downstream of AAT encoding Asn at position 313 of Mdh, respectively. The primer SEQ ID NO 46 was designed by inserting two stop codons (TAGTAG) between GCG encoding Ala at position 312 and AAT encoding Asn at position 313. PCR was performed using Pyrobest(R) DNA polymerase (Takara Bio), and reaction conditions followed the vendor's recommended protocol. After agarose gel electrophoresis of the each amplified approximately 1 kbp DNA fragments, the bands of interest were cut out and collected them from the gel using the Wizard(R) SV Gel and PCR Clean-Up System (Promega). The two DNA fragments collected were inserted into the SmaI site of pBS5T as described in WO2006/057450 by an infusion reaction to obtain the vector pBS5Ts2278 for insertion of the stop codon into the mdh gene. The In-Fusion(R) HD Cloning Kit (Takara Bio) was used for the infusion reaction and the reaction conditions followed the protocol recommended by the supplier.

### (2) Construction of a YDK010::phoS (W302C) strain with a stop codon insertion into the mdh gene.

The YDK010::phoS(W302C) strain described in WO2016/171224 was transformed with pBS5Ts2278 constructed in Example 2(1). From the resulting transformant, a strain was selected according to the method described in WO2006/057450 and the YDK010::phoS(W302C)::s2278 strain with a stop codon inserted in the mdh gene was obtained.

Example 3: Secretory expression of Protein L using Corynebacterium glutamicum lacking the mdh gene.

The plasmid pPK4_CspAss_ProteinL for secretory expression of Protein L described in JP 2016-206702 was used to transform the
YDK010::phoS(W302C) strain and the YDK010::phoS(W302C)Δ2278 strain obtained in Example 1(2) to obtain
YDK010::phoS(W302C)/pPK4_CspAss_ProteinL and
YDK010::phoS(W302C)Δ2278/pPK4_CspAss_ProteinL strains, respectively.

Each resulting transformant was grown in MMTG liquid medium (120 g glucose, 3 g magnesium sulphate heptahydrate, 30 g ammonium sulphate, 1.5 g potassium dihydrogen phosphate, 0.03 g iron sulphate heptahydrate, 0.03 g manganese sulphate pentahydrate, 0.45 mg thiamine HCl, 0.45 mg biotin, 0.15 g DL-methionine, soy hydrochloric acid hydrolysate (0.2 g total nitrogen), 50 g calcium carbonate, adjusted to pH 7.0 in 1 L with water) containing 25 mg/l kanamycin at 30°C for 72 h, respectively.

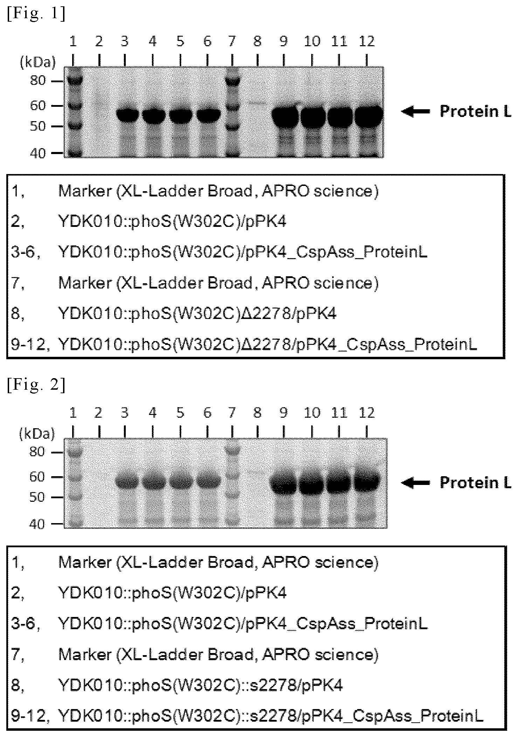

After the end of the culture, 3.0 µl of the culture supernatant obtained by centrifugation of each culture was subjected to reducing SDS-PAGE before staining with SYPRO Ruby (Life technologies). The results showed that Protein L secretion was significantly enhanced in the YDK010::phoS(W302C)Δ2278 strain compared with the YDK010::phoS(W302C) strain (Fig. 1).

After staining, Protein L band intensities were quantified using image analysis software Multi Gauge (FUJIFILM), and the mean value of band intensity when Protein L was expressed in YDK010::phoS(W302C)Δ2278 strain was calculated as the relative value when the mean value of band intensity when Protein L was expressed in YDK010::phoS(W302C) strain was set to 1.00. The results showed that Protein L secretion was enhanced by approximately 2.30-fold in the YDK010::phoS(W302C)Δ2278 strain compared with the YDK010::phoS(W302C) strain (Table 1).

This indicates that the Δmdh mutation (loss of the mdh gene) is an effective mutation resulting in increased secretion in the production of Protein L using the CspA secretion signal in the Sec system.

**Table 1**

| Strain | Relative intensity |
|---|---|
| YDK010::phoS (W 302C)/pPK4_CspAss_ProteinL | 1.00 |
| YDK010::phoS (W 302C) Δ2278/pPK4_CspAss_ProteinL | 2.30 |

Example 4: Secretory expression of Protein L using Corynebacterium glutamicum with a stop codon inserted into the mdh gene.

The plasmid pPK4_CspAss_ProteinL for secretory expression of Protein L was used to transform the YDK010::phoS(W302C) strain and the YDK010::phoS(W302C)::s2278 strain obtained in Example 2(2), to obtain the YDK010::phoS( W302C)/pPK4_CspAss_ProteinL strain and YDK010::phoS(W302C)::s2278/pPK4_CspAss_ProteinL strain, respectively.

Each resulting transformant was grown in MMTG liquid medium (120 g glucose, 3 g magnesium sulphate heptahydrate, 30 g ammonium sulphate, 1.5 g potassium dihydrogen phosphate, 0.03 g iron sulphate heptahydrate, 0.03 g manganese sulphate pentahydrate, 0.45 mg thiamine HCl, 0.45 mg biotin, 0.15 g DL-methionine, soy hydrochloric acid hydrolysate (0.2 g total nitrogen), 50 g calcium carbonate, adjusted to pH 7.0 in 1 L with water) containing 25 mg/l kanamycin at 30°C for 72 h, respectively.

After the end of the culture, 3.0 µl of the culture supernatant obtained by centrifugation of each culture was subjected to reducing SDS-PAGE before staining with SYPRO Ruby (Life technologies). The results showed that Protein L secretion was significantly enhanced in the YDK010::phoS(W302C)::s2278 strain compared with the YDK010::phoS(W302C) strain (Fig. 2).

After staining, Protein L band intensities were quantified using image analysis software Multi Gauge (FUJIFILM), and the mean value of band intensity when Protein L was expressed in the YDK010::phoS(W302C)::s2278 strain was calculated as the relative value when the mean value of band intensity when Protein L was expressed in the YDK010::phoS(W302C ) strain was set to 1.00. The results showed that Protein L secretion was enhanced by approximately 2.78-fold in the YDK010::phoS(W302C)::s2278 strain compared with the YDK010::phoS(W302C) strain (Table 2).

This indicates that not only the Δ2278 mutation (deletion of the mdh gene) but also the s2278 mutation (insertion of a stop codon into the mdh gene) is an effective mutation for increased secretion in the production of Protein L using the CspA secretion signal in the Sec system. The insertion of the stop codon into the mdh gene disrupted the mdh gene, resulting in the same traits as the mdh gene-deficient strain.

**Table 2**

| Strain | Relative intensity |
|---|---|
| YDK010::phoS (W 302C)/pPK4_CspAss_ProteinL | 1.00 |
| YDK010::phoS (W 302C)::s2278/pPK4_CspAss_ProteinL | 2.78 |

Example 5: Secretory expression of Liver-type Fatty acid-binding protein (LFABP) using Corynebacterium glutamicum with a stop codon inserted in the mdh gene.

The pPK4_CspB6Xa-LFABP described in WO2016/171224 was used to transform the YDK010::phoS(W302C) strain and the YDK010::phoS(W302C)::s2278 strain obtained in Example 2(2), to obtain the YDK010::phoS(W The pPK4_CspB6Xa-LFABP strain and the YDK010::phoS(W302C)::s2278/pPK4_CspB6Xa-LFABP strain, respectively. The pPK4_CspB6Xa-LFABP is a plasmid for the secretory expression of human Liver-type fatty acid-binding protein (hereafter referred to as LFABP). pPK4_CspB6Xa-LFABP has the promoter of the cspB gene (PS2 gene) from C. glutamicum ATCC13869 strain, and a gene encoding the 30 amino acid residues of CspB signal peptide from C. glutamicum ATCC13869,the N-terminal 6 amino acid residues of the CspB mature protein from the same strain, the IEGR recognition sequence of the Factor Xa protease, and the LFABP fusion protein (hereafter referred to as CspB6Xa-LFABP) expressibly linked downstream of the promoter.

Each resulting transformant was grown in MMTG liquid medium (120 g glucose, 3 g magnesium sulphate heptahydrate, 30 g ammonium sulphate, 1.5 g potassium dihydrogen phosphate, 0.03 g iron sulphate heptahydrate, 0.03 g manganese sulphate pentahydrate, 0.45 mg thiamine HCl, 0.45 mg biotin, 0.15 g DL-methionine, soy hydrochloric acid hydrolysate (0.2 g total nitrogen), 50 g calcium carbonate, adjusted to pH 7.0 in 1 L with water) containing 25 mg/l kanamycin at 30°C for 72 h, respectively.

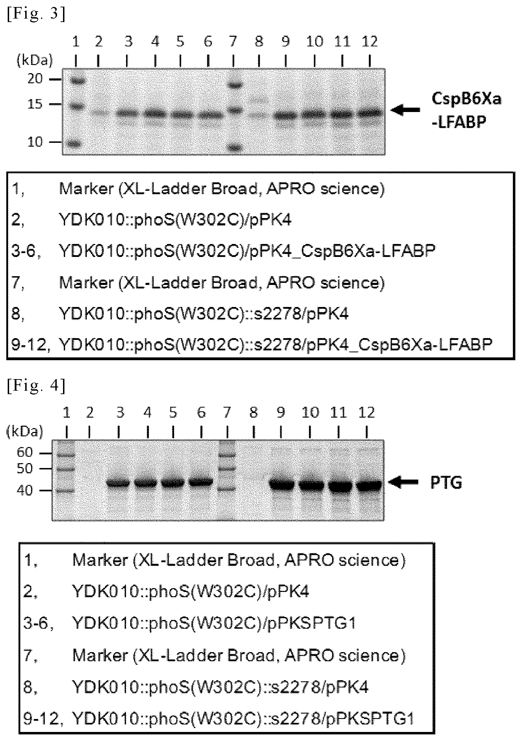

After the end of the culture, 2.0 µl of the culture supernatant obtained by centrifugation of each culture was subjected to reducing SDS-PAGE before staining with SYPRO Ruby (Life technologies). The results showed that the YDK010::phoS(W302C)::s2278 strain had increased secretion amount of CspB6Xa-LFABP compared to the YDK010::phoS(W302C) strain (Fig. 3).

After staining, the band intensity of CspB6Xa-LFABP was quantified using the image analysis software Multi Gauge (FUJIFILM) and the mean value of band intensity when CspB6Xa-LFABP was expressed in the
YDK010::phoS(W302C)::s2278 strain was calculated as the relative value when the mean value of band intensity when a phoS(W302C) strain expressing CspB6Xa-LFABP was set to 1.00. The results showed that CspB6Xa- LFABP secretion was improved by approximately 1.53-fold in the
YDK010::phoS(W302C)::s2278 strain compared with the
YDK010::phoS(W302C) strain (Table 3).

This indicates that disruption of the mdh gene is also an effective mutation resulting in increased secretion amount of CspB6Xa-LFABP during its secretory production in the YDK010::phoS(W302C) strain.

**Table 3**

| Strain | Relative intensity |
|---|---|
| YDK010::phoS (W 302C)/pPK4_CspB6Xa-LFABP | 1.00 |
| YD K010::phoS (W 302C)::s2278/pPK4_CspB6Xa-LFABP | 1.53 |

Example 6: Secretory expression of protransglutaminase from the Sec secretory pathway using Corynebacterium glutamicum with a stop codon inserted in the mdh gene.

The pPKSPTG1 described in WO2001/23591 was used to transform the YDK010::phoS(W302C) strain and the YDK010::phoS(W302C)::s2278 strain obtained in Example 2(2), to obtain the YDK010::phoS(W302C)/pPKSPTG 1 and YDK010::phoS(W302C)::s2278/pPKSPTG1 strains, respectively. pPKSPTG1 is a plasmid for secretory expression of pro-transglutaminase (transglutaminase with pro-structured part; hereafter referred to as PTG) from S. mobaraense. pPKSPTG1 contains the promoter of the cspB gene (PS2 gene) from C. glutamicum ATCC13869 strain and a gene encoding a fusion protein of 25 amino acid residues of signal peptide of CspA (SlpA; Genbank Accession No. BAB62413) from C. ammoniagenes ATCC6872 strain and PTG expressionally linked downstream of the promoter.

Each resulting transformant was grown in MMTG liquid medium (120 g glucose, 3 g magnesium sulphate heptahydrate, 30 g ammonium sulphate, 1.5 g potassium dihydrogen phosphate, 0.03 g iron sulphate heptahydrate, 0.03 g manganese sulphate pentahydrate, 0.45 mg thiamine HCl, 0.45 mg biotin, 0.15 g DL-methionine, soy hydrochloric acid hydrolysate (0.2 g total nitrogen), 50 g calcium carbonate, adjusted to pH 7.0 in 1 L with water) containing 25 mg/l kanamycin at 30°C for 72 h, respectively.

After the end of the culture, 2.5 µl of the culture supernatant obtained by centrifugation of each culture was subjected to reducing SDS-PAGE before staining with SYPRO Ruby (Life technologies). The results showed that PTG secretion was enhanced in the YDK010::phoS(W302C)::s2278 strain compared to the YDK010::phoS(W302C) strain (Fig. 4).

After staining, the band intensity of PTG was quantified using the image analysis software Multi Gauge (FUJIFILM) and the mean value of band intensity when PTG was expressed in the YDK010::phoS(W302C)::s2278 strain was calculated as relative value when the mean value of band intensity when PTG was expressed in the YDK010::phoS (W302C) strain was set to 1.00. The results showed that the YDK010::phoS(W302C)::s2278 strain had an approximately 1.86-fold increase in PTG secretion compared to the YDK010::phoS(W302C) strain (Table 4).

This indicates that disruption of the mdh gene is also an effective mutation resulting in increased secretion amount of PTG during its secretory production the YDK010::phoS(W302C) strain.

**Table 4**

| Strain | Relative intensity |
|---|---|
| YDK010::phoS (W 302C)/pPKSPTG1 | 1.00 |
| YDK010::phoS (W 302C)::s2278/pPKSPTG1 | 1.86 |

Example 7: Secretory expression of protransglutaminase from the Tat secretion pathway using Corynebacterium glutamicum with a stop codon inserted in the mdh gene.

The pPK6_T_PTG described in WO2016/171224 was used to transform the YDK010::phoS(W302C) strain and the YDK010::phoS(W302C)::s2278 strain obtained in Example 2(2), to obtain the YDK010::phoS(W302C)/pPK6 _T_PTG and YDK010::phoS(W302C)::s2278/pPK6_T_PTG strains, respectively. pPK6_T_PTG is a co-expression plasmid for the TatABC secretion machinery and a pro-transglutaminase (transglutaminase with a pro-structure part; hereafter referred to as PTG) from S. mobaraense. pPK6_T_PTG has the promoter of the cspB gene (PS2 gene) from C. glutamicum ATCC13869, and a gene encoding a fusion protein of the TorA signal peptide from E. coli and PTG expressibly linked downstream of the promoter.

Each resulting transformant was grown in MMTG liquid medium (120 g glucose, 3 g magnesium sulphate heptahydrate, 30 g ammonium sulphate, 1.5 g potassium dihydrogen phosphate, 0.03 g iron sulphate heptahydrate, 0.03 g manganese sulphate pentahydrate, 0.45 mg thiamine HCl, 0.45 mg biotin, 0.15 g DL-methionine, soy hydrochloric acid hydrolysate (0.2 g total nitrogen), 50 g calcium carbonate, adjusted to pH 7.0 in 1 L with water) containing 25 mg/l kanamycin at 30°C for 72 h, respectively.

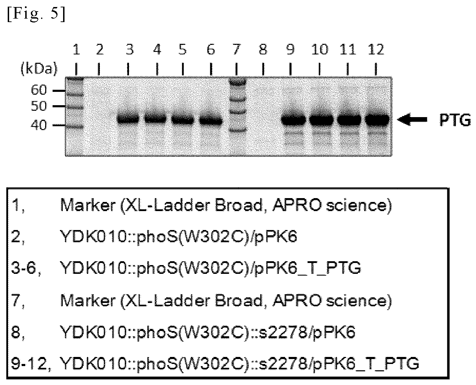

After the end of the culture, 1.0 µl of the culture supernatant obtained by centrifugation of each culture was subjected to reducing SDS-PAGE before staining with SYPRO Ruby (Life technologies). The results showed that PTG secretion was significantly enhanced in the YDK010::phoS(W302C)::s2278 strain compared with the YDK010::phoS(W302C) strain (Fig. 5).

After staining, the band intensity of PTG was quantified using the image analysis software Multi Gauge (FUJIFILM) and the mean value of band intensity when PTG was expressed in the YDK010::phoS(W302C)::s2278 strain was calculated as the relative value when the mean value of band intensity when PTG was expressed in the YDK010::phoS(W302C) strain was set at 1.00. The results showed that the YDK010::phoS(W302C)::s2278 strain had an approximately 1.83-fold increase in PTG secretion compared to the YDK010::phoS(W302C) strain (Table 5).

This indicates that disruption of the mdh gene is also an effective mutation resulting in increased secretion amount of PTG during its secretory production using the TorA signal sequence in the YDK010::phoS(W302C) strain. In other words, disruption of the mdh gene was found to be a mutation that can significantly improve the secretion of a heterologous protein not only in the Sec secretion pathway, but also in the Tat secretion pathway.

**Table 5**

| Strain | Relative intensity |
|---|---|
| YDK010::phoS (W 302C)/pPKSPTG1 | 1.00 |
| YDK010::phoS (W 302C)::s2278/pPKSPTG1 | 1.86 |

### <Explanation of Sequence Listing>

SEQ ID NO 1: nucleotide sequence of the phoS gene of C. glutamicum YDK010.
SEQ ID NO 2: amino acid sequence of the PhoS protein of C. glutamicum YDK010.
SEQ ID NO 3: amino acid sequence of the PhoS protein of C. glutamicum ATCC 13032.
SEQ ID NO 4: amino acid sequence of the PhoS protein of C. glutamicum ATCC 14067.
SEQ ID NO 5: amino acid sequence of the PhoS protein of C. callunae.
SEQ ID NO 6: amino acid sequence of the PhoS protein of C. crenatum.
SEQ ID NO 7: amino acid sequence of the PhoS protein of C. efficiens.
SEQ ID NO 8: nucleotide sequence of the phoR gene of C. glutamicum ATCC 13032.
SEQ ID NO 9: amino acid sequence of the PhoR protein of C. glutamicum ATCC 13032.
SEQ ID NO 10: nucleotide sequence of the cspB gene of C. glutamicum ATCC 13869.
SEQ ID NO 11: amino acid sequence of the CspB protein of C. glutamicum ATCC 13869.
SEQ ID NO 12: nucleotide sequence of the tatA gene of C. glutamicum ATCC 13032.
SEQ ID NO 13: amino acid sequence of the TatA protein of C. glutamicum ATCC 13032.
SEQ ID NO 14: nucleotide sequence of the tatB gene of C. glutamicum ATCC 13032.
SEQ ID NO 15: amino acid sequence of the TatB protein of C. glutamicum ATCC 13032.
SEQ ID NO 16: nucleotide sequence of the tatC gene of C. glutamicum ATCC 13032.
SEQ ID NO 17: amino acid sequence of the TatC protein of C. glutamicum ATCC 13032.
SEQ ID NO 18: amino acid sequence of the TorA signal peptide.
SEQ ID NO 19: amino acid sequence of the SufI signal peptide.
SEQ ID NO 20: amino acid sequence of the PhoD signal peptide.
SEQ ID NO 21: amino acid sequence of the LipA signal peptide.
SEQ ID NO 22: Amino acid sequence of the IMD signal peptide.
SEQ ID NO 23: amino acid sequence of the twin arginine motif.
SEQ ID NO 24: Skipped sequence.
SEQ ID NO 25: amino acid sequence of the PS1 signal peptide.
SEQ ID NO 26: amino acid sequence of PS2 signal peptide.
SEQ ID NO 27: amino acid sequence of the SlpA signal peptide.
SEQ ID NO 28: amino acid sequence of the CspB mature protein of C. glutamicum ATCC 13869.
SEQ ID NOs 29-31: skipped sequences.
SEQ ID NOs 32-36: amino acid sequence of one of the insertion sequences used in the present invention.
SEQ ID NO 37: Recognition sequence of Factor Xa protease.
SEQ ID NO 38: Recognition sequence of ProTEV protease.
SEQ ID NO 39: nucleotide sequence of the mdh gene of C. glutamicum ATCC 13869.
SEQ ID NO 40: amino acid sequence of the Mdh protein of C. glutamicum ATCC 13869.
SEQ ID NOs 41-48: primers.

## Claims

1. A method for producing a heterogeneous protein, comprising:
culturing a coryneform bacterium having a genetic construct for a secretory expression of the heterologous protein; and
collecting the heterologous protein secretory produced,
wherein said coryneform bacterium has been modified so that an activity of a Mdh protein is reduced as compared to an unmodified bacterium,
wherein the genetic construct contains, in the direction from 5' to 3', a promoter sequence that functions in the coryneform bacteria, a nucleic acid sequence encoding a signal peptide that functions in the coryneform bacteria, and a nucleic acid sequence encoding the heterologous protein, and
wherein the heterologous protein is expressed as a fusion protein with the signal peptide.

2. The method according to claim 1, wherein the Mdh protein is a protein as described in (a), (b) or (c) below:
(a) a protein comprising the amino acid sequence of SEQ ID NO 40;
(b) a protein comprising the amino acid sequence of SEQ ID NO 40 but which includes a substitution(s), a deletion(s), an insertion(s) and/or an addition(s) of 1 to 10 amino acid residues, and having a malate dehydrogenase activity; and
(c) a protein comprising an amino acid sequence having 90% or more identity to the amino acid sequence of SEQ ID NO 40 and having the malate dehydrogenase activity.

3. The method according to claim 1 or 2, wherein the activity of the Mdh protein is reduced by decreasing the expression of a mdh gene or by disrupting a mdh gene.

4. The method according to claim 1 or 2, wherein the activity of the Mdh protein is reduced due to a deletion of part or all of the amino acid sequence of the Mdh protein.

5. The method according to claim 4, wherein at least the region corresponding to positions 313 to 328 of SEQ ID NO 40 in the amino acid sequence of the Mdh protein is deleted.

6. The method according to claim 4, wherein at least 16 residues at the C-terminus of the amino acid sequence of the Mdh protein are deleted.

7. The method according to claim 4, wherein the deletion was caused by deletion of part or all of the coding region of the mdh gene, an introduction of a stop codon into the coding region of the mdh gene, a frameshift in the coding region of the mdh gene, or a combination thereof.

8. The method according to claim 1 or 2, wherein the coryneform bacterium is further modified to harbor a phoS gene encoding a mutated PhoS protein.

9. The method according to claim 8, wherein the mutation is a replacement of an amino acid residue corresponding to the tryptophan residue at position 302 of SEQ ID NO 2 in the wild-type PhoS protein with an amino acid residue other than aromatic amino acids and histidine residues.

10. The method according to claim 9, wherein the amino acid residue other than aromatic amino acids and histidine residues is a lysine residue, alanine residue, valine residue, serine residue, cysteine residue, methionine residue, aspartic acid residue or aspartic residue.

11. The method according to claim 9, wherein the wild-type PhoS protein is a protein as described in (a), (b) or (c) below:
(a) a protein comprising any of the amino acid sequences of SEQ ID NOs 2 to 7;
(b) a protein comprising any of the amino acid sequences of SEQ ID NOs 2 to 7, but which includes a substitution(s), a deletion(s), an insertion(s) and/or an addition(s) of 1 to 10 amino acid residues, and having a function as a sensor kinase of the PhoRS system; and
(c) a protein comprising an amino acid sequence having 90% or more identity to any of the amino acid sequences of SEQ ID NOs 2 to 7 and having a function as a sensor kinase of the PhoRS system.

12. The method according to claim 1 or 2, wherein the signal peptide is a Tat-dependent signal peptide.

13. The method according to claim 12, wherein the Tat-dependent signal peptide is selected from the group consisting of a TorA signal peptide, SufI signal peptide, PhoD signal peptide, LipA signal peptide and IMD signal peptide.

14. The method according to claim 12, wherein the coryneform bacterium is further modified so that the expression of one or more genes encoding a Tat secretion system is increased as compared to an unmodified bacterium.

15. The method according to claim 14, wherein the genes encoding the Tat secretion system comprise a tatA gene, tatB gene, tatC gene and tatE gene.

16. The method according to claim 1 or 2, wherein the signal peptide is a Sec-dependent signal peptide.

17. The method according to claim 16, wherein the Sec-dependent signal peptide is a signal peptide selected from the group consisting of a PS1 signal peptide, PS2 signal peptide and SlpA signal peptide.

18. The method according to claim 1 or 2, wherein the genetic construct further comprises a nucleic acid sequence encoding an amino acid sequence comprising Gln-Glu-Thr between the nucleic acid sequence encoding the signal peptide that functions in the coryneform bacteria and the nucleic acid sequence encoding the heterologous protein.

19. The method according to claim 18, wherein the genetic construct further comprises a nucleic acid sequence encoding an amino acid sequence used for an enzymatic cleavage between the nucleic acid sequence encoding the amino acid sequence comprising Gln-Glu-Thr and the nucleic acid sequence encoding the heterologous protein.

20. The method as in claim 1 or 2, wherein the coryneform bacterium belongs to the genus *Corynebacterium.*

21. The method according to claim 20, wherein the coryneform bacterium is *Corynebacterium glutamicum.*

22. The method according to claim 21, wherein the coryneform bacterium is a modified strain derived from *Corynebacterium glutamicum* AJ12036 (FERM BP-734) or a modified strain derived from *Corynebacterium glutamicum* ATCC 13869.

23. The method according to claim 1 or 2, wherein the coryneform bacterium is a coryneform bacterium having a reduced number of molecules per cell of cell surface proteins as compared to the unmodified bacterium.
